# EUROPEAN PATENT SPECIFICATION

(11) **EP 1 434 602 B1**
(45) Date of publication and mention of the grant of the patent: **17.12.2014**
(21) Application number: 02782841.7
(22) Date of filing: 07.10.2002
(51) Int. Cl.: A61K 39/39, A61K 31/7088, A61K 9/107, A61P 31/00, A61P 35/00

(54) **CpG plus oil in water emulsion as adjuvant system for truncated bovine herpesvirus-1 glycoprotein D**
CpG plus Öl in Wasser Emulsionen als Adjuvantsystem fuer trunkiertes bovines Herpesvirus-1 Glykoprotein D
CpG plus huile en eau émulsions adjuvants pour herpèsvirus bovin de type 1 glycoprotéine D tronquéeÜ

(30) Priority: 06.10.2001 US 327734 P
(43) Date of publication of application: 07.07.2004
(73) Proprietor: Merial Limited, Duluth, GA 30096 (US); UNIVERSITY OF SASKATCHEWAN, Saskatoon, Saskatchewan S7N 5C8 (CA)
(72) Inventor: BABIUK, Lorne, Alan, Saskatoon, Saskatchewan S7J 2Y1 (CA); HECKER, Rolf, 40597 Düsseldorf (DE)
(74) Representative: Mallalieu, Catherine Louise
(86) International application number: PCT/EP2002/011206
(87) International publication number: WO 2003/030934

(56) References cited:
- WO-A-00/50075
- WO-A-01/68103
- WO-A-98/40100
- US-A- 5 951 988
- US-B1- 6 610 308
- IOANNOU X P ET AL: "The immunogenicity and protective efficacy of bovine herpesvirus 1 glycoprotein D plus emulsigen are increased by formulation with CpG oligodeoxynucleotides." JOURNAL OF VIROLOGY, vol. 76, no. 18, September 2002 (2002-09), pages 9002-9010, XP002247542 September, 2002 ISSN: 0022-538X
- SINGH M ET AL: "Cationic microparticles are an effective delivery system for immune stimulatory cpG DNA.", PHARMACEUTICAL RESEARCH OCT 2001 LNKD- PUBMED:11697476, vol. 18, no. 10, October 2001 (2001-10), pages 1476-1479, ISSN: 0724-8741
- VAN DRUNEN LITTEL-VAN DEN HURK S ET AL: "Recent advances in the use of DNA vaccines for the treatment of diseases of farmed animals.", ADVANCED DRUG DELIVERY REVIEWS 15 SEP 2000 LNKD- PUBMED:10967218, vol. 43, no. 1, 15 September 2000 (2000-09-15), pages 13-28, ISSN: 0169-409X
- RANKIN R ET AL: "CpG motif identification for veterinary and laboratory species demonstrates that sequence recognition is highly conserved.", ANTISENSE & NUCLEIC ACID DRUG DEVELOPMENT OCT 2001 LNKD- PUBMED:11763350, vol. 11, no. 5, October 2001 (2001-10), pages 333-340, ISSN: 1087-2906
- KRIEG A M ET AL: "Enhancing vaccines with immune stimulatory CpG DNA.", CURRENT OPINION IN MOLECULAR THERAPEUTICS FEB 2001 LNKD- PUBMED:11249727, vol. 3, no. 1, February 2001 (2001-02), pages 15-24, ISSN: 1464-8431

## Description

### Field of the Invention

The present invention relates to the use of immunostimulatory nucleic acids in combination with other therapeutic formulations.

### Background of the Invention

In United States alone the death rate due to infectious disease rose 58 % between 1980 and 1992. During this time, the use of anti-infective therapies to combat infectious disease has grown significantly and is now a multi-billion dollar a year industry. Even with these increases in anti-infective agent use, the treatment and prevention of infectious disease remains a challenge to the medical community throughout the world. In general, there are three types of anti-infective agents, anti-bacterial agents, anti-viral agents, and anti-fungal agents, and even within these classes of agents there is some overlap with respect to the type of microorganism they are useful for treating.

One of the problems with anti-infective therapies is the side effects occurring in the host that is treated with the anti-infective. For instance, many anti-infectious agents can kill or inhibit a broad spectrum of microorganisms and are not specific for a particular type of species. Treatment with these types of anti-infectious agents results in the killing of the normal microbial flora living in the host, as well as the infectious microorganism. The loss of the microbial flora can lead to disease complications and predispose the host to infection by other pathogens, since the microbial flora compete with and function as barriers to infectious pathogens. Other side effects may arise as a result of specific or non-specific effects of these chemical entities on non-microbial cells or tissues of the host.

In addition to anti-infective agents, vaccines are used to prevent and treat infectious disease. Vaccines include an antigen in combination with an adjuvant. Adjuvants play an important role in the efficacy of vaccines of the treatment and prevention of infectious disease. In addition to increasing the strength and kinetics of an immune response, adjuvants also play a role in determining the type of immune response generated. Aluminum compounds, including aluminum hydroxide and aluminum phosphate, are widely used with human vaccines. These adjuvants skew the immune response towards a T-helper type 2 (Th2) response, which is characterized by the secretion of Th2 type cytokines such as IL-4 and IL-5 and the generation of IgG1 and IgE type antibodies, but weak or absent cytotoxic T lymphocyte (CTL) responses *(*Bomford, R. 1998. Will adjuvants be needed for vaccines of the future? Dev.Biol.Stand. 92:13-17*;* Brazolot Millan, C.L., et al. 1998. CpG DNA can induce strong Th1 humoral and cell-mediated immune responses againsthepatitis B surface antigen in young mice. Proc.Natl.Acad.Sci.USA 95:15553-15558*;* Davis, H.L., et al. 1998. CpG DNA is a potent enhancer of specific immunity in mice immunized withrecombinant hepatitis B surface antigen. J.Immunol 160:870-876*)*. Development of the appropriate type of immune response is essential for successful immunization. Strong innate immunity that is associated with a Th1 type immune response, is thought to be essential for the control of intracellular pathogens, whereas strong humoral immunity, which can be found with both Th1 and Th2 type immune responses, appears to be essential for the control of extracellular pathogens *(*Constant, S.L. and K. Bottomly. 1997. Induction of Th1 and Th2 CD4+ T cell responses: the alternative approaches. Ann.Rev.Immunol. 15:297-322*).* Synthetic oligodeoxynucleotides containing unmethylated CpG dinucleotides (CpG ODN) are novel adjuvants known to promote Th1 type immune responses with the secretion of IFN-γ, TNF-α and IL-12 cytokines, opsonizing antibodies such as those of the IgG2a isotype, and strong CTL induction *(*Chu, R.S., et al. 1997. CpG oligodeoxynucleotides act as adjuvant that switch on T helper 1 (Th1)immzmity. J.Exp.Med 186:1623-1631*;* Klinman, D.M et al. 1999. CpG motifs as immune adjuvants. Vaccine 17:19-25*).*

Bovine herpesvirus-1 (BHV-1), a member of the alphaherpesvirinae subfamily, is associated with a variety of clinical disease manifestations including rhinotracheitis, vulvovaginitis, abortions, conjuctivitis, encephalitis and generalized systemic infections *(*Gibbs, E.P.J. and M.M. Rweyemamu. 1977. Bovine herpesvirus-1, p. 317AnonymousBovine herpesvirus. Vet. Bull, London *;* Yates, W.D. G. 1982. A review of infectious bovine rhinotracheitis, shipping fever pneumonia and viral-bacterial synergism in respiratory disease of cattle. Can.J.Comp.Med. 46:225-263*).* Bovine respiratory diseases cost the cattle industry up to $1 billion per year in North America *(*Yates, W.D.G. 1982. A review of infections bovine rhinotracheitis, shipping fever pneumonia and viral-bacterial synergism in respiratory disease of cattle. Can.J. Comp.Med. 46:225-263*).* These losses occur even though live attenuated and killed vaccines are available. At present, the greatest potential for combined efficacy, safety, antigenic specificity and protection against BHV-1 resides in subunit vaccines consisting of one or more of the viral glycoproteins, gB, gC and gD and an adjuvant Conventional adjuvants such as VSA3, however, not only generate a Th2-like immune response, but are not metabolized and leave injection site reactions. Such reactions are unacceptable for human or veterinary vaccines.

WO 01/68103 discloses methods for preventing and/or treating herpes virus infection comprising administering a composition comprising an immunostimmulatory sequence comprising the sequence 5'-C, G-3'. The immunostimulatory sequence is administered without an *alphaherpesvirinae* antigen.

WO 00/50075 teaches an immunogenic composition comprising an immunostimulating amount of a Neisseria antigen and an immunostimmulatory amount of an adjuvant composition comprising an oligonucleotide comprising at least one CG motif.

### Summary of the Invention

The scope of the present invention is defined by the claims and any information that does not fall within the claims is provided for information only.

The invention provides improved methods and products for the treatment of subjects using immunostimulatory nucleic acids in combination with specific formulations. The invention is based, in part, on the finding that when some types of immunostimulatory nucleic acid molecules are used in conjunction with specific formulations, some unexpected and improved results are observed. For instance, the efficacy of the combination of some immunostimulatory nucleic acids and the formulation is profoundly improved over the use of the immunostimulatory nucleic acid alone. The results are surprising, in part, because the immunostimulatory nucleic acids and the formulations act through different mechanisms and would not necessarily be expected to improve the efficacy of the other in a synergistic manner.

In one aspect the invention relates to a method for reducing viral shedding in a non-human animal by administering to a non-human animal infected with a bovine herpesvirus-1 or at risk of infection with bovine herpesvirus-1, an immunostimulatory nucleic acid and an oil-in-water emulsion in an effective amount to reduce viral shedding. In one embodiment the oil-in-water emulsion is EMULSIGEN^{™}. Optionally the non-human animal is a dog, cat, horse, cow, pig, sheep, goat, primate or chicken.

The combination of active agents may be administered with an antiviral agent. In some embodiments the antiviral agent is selected from the group consisting of Acemarman; Acyclovir, Acyclovir Sodium; Adefovir; Alovudine; Alvircept Sudotox; Amantadine Hydrochloride; Aranotin; Arildone; Atevirdine Mesylate; Avridine; Cidofovir, Cipamfylline; Cytarabine Hydrochloride; Delavirdine Mesylate; Desciolovir; Didanosine; Disoxaril; Edoxudine; Enviradene; Enviroxime; Famciclovir; Famotins Hydroohloride; Fiacitabine; Fialurdine; Fosarilate; Foscarnet Sodium; Fosfonet Sodium; Ganciclovir; Cranciclovir Sodium; Idoxuridine; Kethoxal; Lamivudine; Lobucavir; Memotine Hydrochloride; Methisazone; Nevirapine; Penciclovir; Pirodavir; Ribavirin; Rimantadine Hydrochloride; Saquinavir Mesylate; Somantadine Hydrochloride; Sorivudine; Statolon; Stavudine; Tilorone Hydrochloride; Trifluridine; Valacyclovir Hydrochloride; Vidarabine; Vidarabine Phosphate; Vidarabine Sodium Phosphate; Viroxime; Zalcitabine; Zidovudine; and Zinviroxime.

In other aspects the invention is a method for reducing tissue damage upon vaccination of a subject by administering to a subject by an invasive route an adjuvanted vaccine and an immunostimulatory nucleic acid in an effective amount to reduce tissue damage arising from the adjuvanted vaccine, wherein the vaccine is adjuvanted with an oil-in-water emulsion. In one embodiment the oil-in-water emulsion is EMULSIGEN^{™}. The invasive route may be any type of route that produces an opening in a tissue barrier, such as skin. In some embodiments the invasive route is subcutaneous or intramuscular.

According to other aspects the invention is a method for inducing an immune response by administering to a subject an oil-in-water emulsion and a CpG oligonucleotide in an effective amount to produce the immune response. Optionally the immune response is an antigen specific immune response and the subject is administered an antigen. In one embodiment the oil-in-water emulsion is EMULSIGEN^{™}.

According to yet other aspects the invention relates to a method for reducing a dosage of antigen administered to a subject to produce an antigen specific immune response by administering to a subject an antigen in a sub-therapeutic dosage and an immunostimulatory nucleic acid, wherein the combination of the sub-therapeutic dose of the antigen and the immunostimulatory nucleic acid produce an antigen specific immune response. In some embodiments the sub-therapeutic dose of the antigen is a dose which is at least 50% less than a minimal effective dose of antigen for producing an antigen specific immune response when the antigen is formulated with alum. Alternatively the sub-therapeutic dose of the antigen may be a dose which is at least 90% less than a minimal effective dose of antigen for producing an antigen specific immune response when the antigen is formulated with alum.

The methods of the invention involve the use of an immunostimulatory nucleic acid. The immunostimulatory nucleic acid may be a CpG oligonucleotide and in some embodiments is 2007 (TCGTCGTTGTCGTTTTGTCGTT); 2142 (TCGCGTGCGTTTTGTCGTTTTGACGTT); 2135 (TCGTCGTTTGTCGTTTTGTCGTT); and/or 2216 (ggGGGACGATCGTCgggggG). Alternatively the immunostimulatory nucleic acid may be a T-rich nucleic acid, such as the ODN of SEQ ID NO: 52 -57 and/or SEQ ID NO: 62-94 or a poly-G nucleic acid such as the ODN of SEQ ID NO: 46, SEQ ID NO: 47, SEQ ID NO: 58, SEQ ID NO: 61, and/or SEQ ID NO: 95-133. In other embodiments the immunostimulatory nucleic acid may have a sequence selected from the group consisting of SEQ ID NO: 1 through to SEQ ID NO: 146.

The immunostimulatory nucleic acid, such as the CpG oligonucleotide may be administered a single time or multiple times. If the CpG oligonucleotide is administered multiple times it may be administered at regular intervals, such as, for example, on a weekly basis, on a daily basis, or on a monthly basis.

The immunostimulatory nucleic acid, such as the CpG oligonucleotide may be administered by any route. For instance the immunostimulatory nucleic acid may be administered orally, by injection, or through a sustained release device.

The immunostimulatory nucleic acid may have a modified backbone, such as a phosphate modified backbone or a peptide modified oligonucleotide backbone. In one embodiment the phosphate modified backbone is a phosphorothioate modified backbone.

The invention is a composition of an immunostimulatory nucleic acid and an oil-in-water emulsion and truncated BHV-1 glycoprotein D. In one embodiment the oil-in-water emulsion is EMULSIGEN^{™}.

In some aspects of the invention, it is conceivable that one or more different CpG immunostimulatory nucleic acids may be administered to a subject. Thus depending on the embodiment, one, two, three, four, five or more different CpG immunostimulatory-nucleic acids may be administered to a subject in a particular method.

According to other embodiments, the immunostimulatory nucleic acid is administered concurrently with, prior to, or following the administration of the other therapeutic formulation, e.g., oil-in-water emulsion, antigen etc.

In some embodiments, the immunostimulatory nucleic acid is administered in an effective amount for upregulating, enhancing or activating an immune response. In some embodiments, the immunostimulatory nucleic acid is administered in an effective amount for redirecting the immune response from a Th2 to a Th1 immune response.

### Brief Description of the Figures

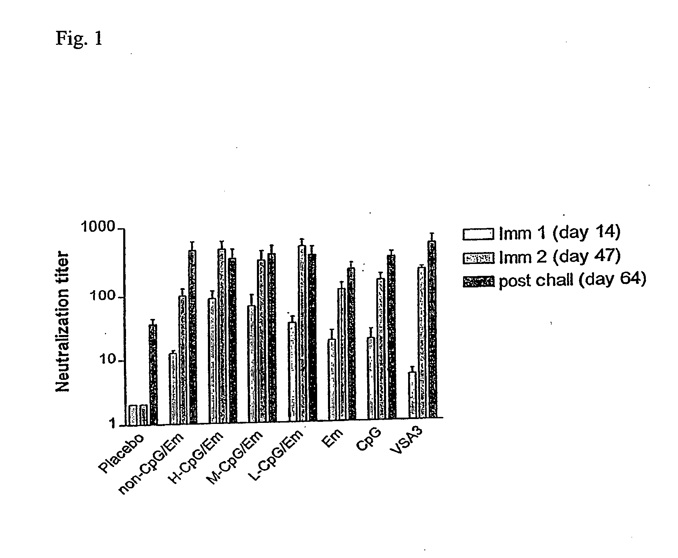

Figure 1 is a bar graph depicting BHV-1 neutralizing antibody responses in the serum of vaccinated and control animals 14, 47 and 64 days after primary immunization (Imm 1 (day 14); 14 days after primary immunization, Imm 2 (day 47); 8 days after secondary immunization, post chall (day 64); 11 days after viral challenge). Antibody titers are expressed as a 50% endpoint using 100 PFU of BHV-1. Error bars show the standard error of the geometric means of seven animals.

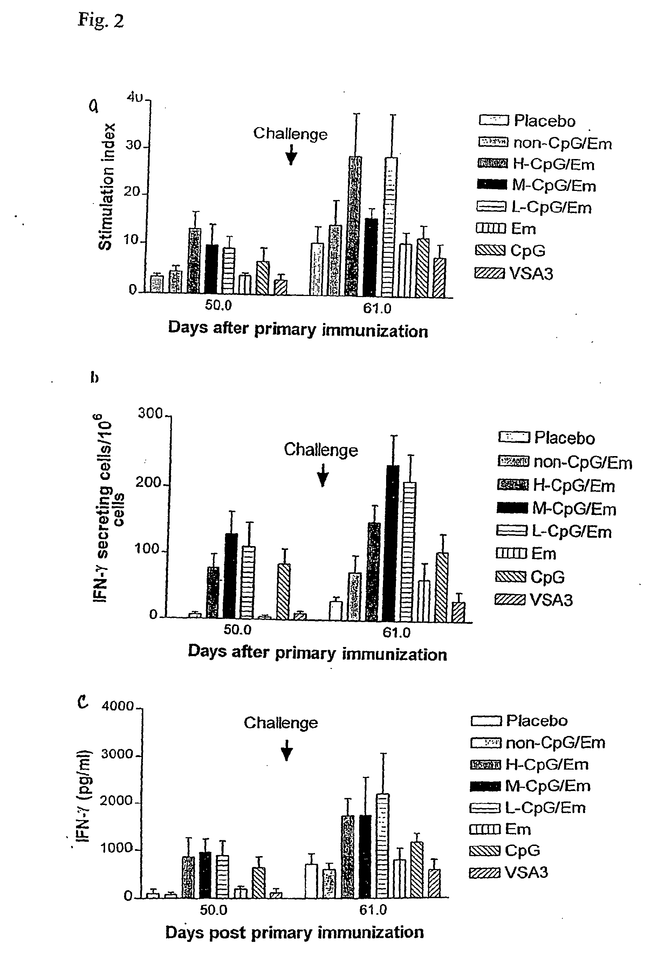

Figure 2 is three bar graphs depicting cellular immune responses after vaccination. Data are expressed as average ± standard error of the mean. (**a**) Antigen-specific proliferation of PBMC before and after challenge. Stimulated index represents the counts per min in the presence of antigen divided by counts per min in the absence of antigen. (**b**) Difference in the number of spots per 10⁶ cells in antigen-stimulated wells and the number of spots per 10⁶ cells in nonstimulated wells. (**c**) Amount of IFN-γ secreted by PBMC in response to BHV-1 gD after 24 hours.

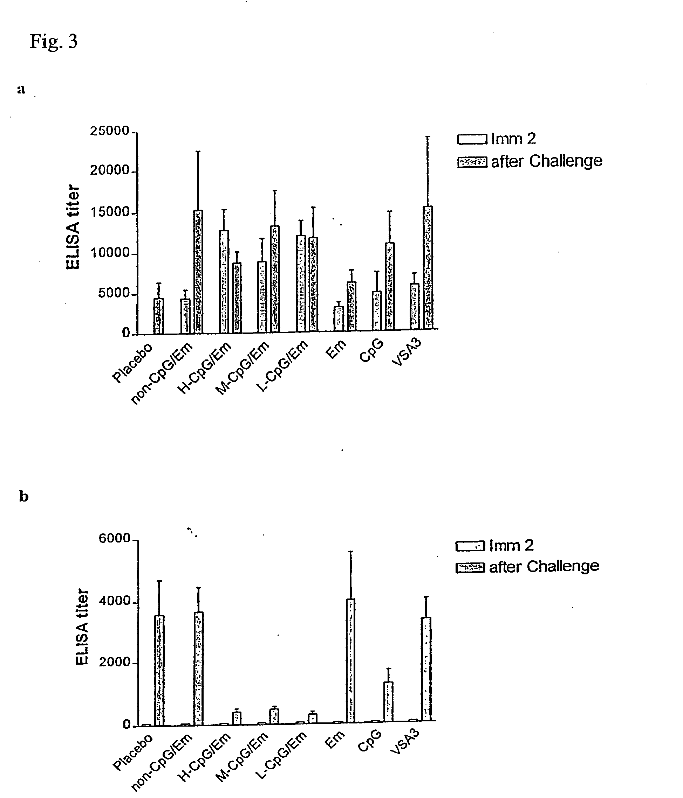

Figure 3 is two bar graphs depicting serum antibodies against BHV-1 glycoproteins 8 days after secondary immunization (Imm 2) and 11 days viral infection (after challenge). (**a**) Antibodies against tgD (**b**) Antibodies against tgB.
Figure 4 is two graphs depicting the effect of immunization on rectal temperature in animals challenged with BHV-1. **a.** mean temperature response **b.** number of fever days; total number of days temperature was ≥40°C.
Figure 5 is two graphs depicting the effect of immunization on weight gain in animals challenged with BHV-1. (**a**) Cumulative weight change. (**b**) Number of animal days weight loss was above of below 5 kg.
Figure 6 is a graph depicting the extent of viral replication following -1 challenge. On the day of challenge and on alternative days thereafter, virus titres were determined in the nasal secretions of immunized animals. Error bars show the standard error of the geometric means of seven animals.

### Detailed Description of the Invention

The scope of the present invention is defined by the claims and any information that does not fall within the claims is provided for information only.

It was surprisingly discovered according to the invention that select combinations of CpG immunostimulatory nucleic acids and oil-in-water emulsions work dramatically better, and sometimes even synergistically, to improve an immune response than either component alone. Although many formulations have been developed and tested for administering drugs, these particular types dramatically enhance the activity of the immunostimulatory nucleic acids. This was surprising, in part, because other similar formulations did not demonstrate the same dramatic types of improvements as the therapeutic formulations described herein. The term "therapeutic formulations" as used herein refers to oil-in-water emulsions such as EMULSIGEN^{™}.

As demonstrated in the Examples described below the combination of immunostimulatory nucleic acids has demonstrated significantly improved therapeutic effects in the treatment and prevention of infectious disease. Recently, it was shown that the combination of CpG ODN with alum had great potential to augment immune responses in mice with minimal side effects at the injection site compared with other adjuvant combinations *(*Weeratna, R.D., et al. 2000. CpG DNA induces stronger immune responses witch less toxicity than other adjuvants. Vaccine 18:1755-1762*).* However, despite the promising results in mice, it was found that BHV-1 tgD adjuvanted with a combination of CpG ODN and alum induced similar immune responses to tgD adjuvanted with CpG ODN alone, and failed to completely protect calves from BHV-1 challenge. In addition, BHV-1 subunit vaccines adjuvanted with Freund's incomplete adjuvant also failed to protect calves from BHV-1 challenge *(*Israel, B.A., et al. 1988. Epitope specificity and protective efficacy of the bovine immune response to bovine herpesvirus-1 glycoprotein vaccines. Vaccine 6:349-356*).*

Surprisingly it has been discovered according to the invention that a truncated BHV-1 tgD vaccine co-adjuvanted with CpG ODN and an oil-in-water emulsion, such as EMULSIGEN^{™} induced a stronger and more balanced immune response as well as provided a greater protection from BHV-1 challenge than tgD adjuvanted with CpG ODN, VSA3 or EMULSIGEN^{™} alone, or co-adjuvanted with a non-CpG ODN and EMULSIGEN^{™}. Furthermore, the immune responses induced by tgD formulated with CpG ODN in the presence or absence of EMULSIGEN^{™} were more Thl-biased in contrast to those formulated with EMULSIGEN^{™}, VSA3 or non-CpG ODN and EMULSIGEN^{™}. The data demonstrates that immunization of animals with a vaccine such as BHV-1 subunit vaccines adjuvanted with CpG ODN and EMULSIGEN^{™}, induces stronger more balanced humoral and cellular responses and a greater protection against viral infection than do vaccines adjuvanted with non-CpG ODN with EMULSIGEN^{™}, EMULSIGEN^{™}, CpG ODN or VSA3 alone.

In cattle, protection against BHV-1 is largely mediated by marked humoral immune responses. Indeed, strong cellular responses in the absence of high antibody titers do not fully protect against infection *(*Loehr, B.L, et al 2000. Gene gun-mediated DNA immunization primers development of mucosal impunity against bovine herpesvirus 1 in cattle. J. Virol. 74:6077-6086*).* Previous studies indicate that significant protection from BHV-1-induced disease can be achieved by subunit vaccines containing one or more of the viral glycoproteins *(* Gao, Y., et al. 1994. Truncated bovine herpesvirus-1 glycoprotein I (gpI) initiates a protective local immune response in its natural host. Vaccine 12:145-152*;* Baca-Estrada, M.E., et al. 1996. Immunogenicity of bovine herpesvirus 1 glycoprotein D in mice: effect of antigen form on the induction of cellular and humoral immune responses. Viral Immunol 9:11-22*;* Babiuk, L.A., L. et al. 1996. Immunology of bovine herpesvirus 1 inflection. Vet.Microbiol. 53:31-42*;* Zhu, X., S. et al. 1997. Yeast-secreted bovine herpesvirus type 1 glycoprotein D has authentic conformational structure and immunogenicity. Vaccine 15:679-688*;* van Drumen Littel-van den Hurk, S., et al. 1994. A subunit gIV vaccine, produced by transfected mammalian cells in culture, induces mucosal immunity against bovine herpesvirus-1 in cattle. Vaccine 12:1295-1302*).* However, due to inefficient humoral immune responses, some BHV-1 subunit vaccines induce little or no protection from challenge *(*Israel, B.A., et al. 1988. Epitope specificity and protective efficacy of the bovine immune response to bovine herpesvirus-1 glycoproteins vaccines. Vaccine 6:349-356*).* Conventional adjuvants such as VSA3 generate strong immune responses but they leave undesirable injection site reactions. VSA3 consists of a mineral oil-based emulsion and an inflammatory compound: dimethyl dioctadecyl ammonium bromide (DDA). In humans, DDA is known to induce a host of inflammatory reactions, including swelling and pain and delayed-type hypersensitivity at the site of injection *(*Vogel, F.R. and M.F. Powell. 1995. A compendium of vaccine adjuvants and excipients, p. 141-228. In M.F. Powell, M.J. Newman, and J.R. Burdman (eds.), Vaccine Design: the subunit and adjuvant approach. Plenum Press, New York*).* Because of its inflammatory tendencies, DDA is also used to induce experimental arthritis in rats *(*Mia, M.Y., Let al. 2000. Dimethyl dioctadecyl ammonium bromide (DDA)-induced arthritis in rats: a model of experimental arthritis. J.Autoimmun. 14: 303-310*).*

Additionally it was discovered that enhanced protective immune responses are induced using the subcutaneous (s.c.) route of delivery. Subcutaneous administration is a useful mode of delivery in both veterinary and human practice because of its ease of administration and the immunocompetence of the skin. Generally vaccines have been administered intramuscularly (i.m.). The compositions of the invention may have even more enhanced effects when delivered s.c. than i.m.

The data presented below demonstrated that the combination of CpG immunostimulatory nucleic acids with the therapeutic formulations resulted in a dramatic decrease in viral shedding. In fact some animals demonstrated zero viral shedding. This is an extremely important parameter because it reflects the amount of protection from infection. "Viral shedding" refers to production of viral particles at a mucosal surface by an animal infected with a virus. The presence or absence of viral shedding can be determined by taking a sample from an animal (i.e., nasal secretions) and analyzing the sample for the presence of virus. If a drug prevents viral shedding it effectively prevents infection in the animal. The ability of the nucleic acids in the therapeutic formulations of the invention to reduce and even eliminate viral shedding demonstrates the surprising potency of the composition.

Thus the immunostimulatory nucleic acids combined with the therapeutic formulations stimulate the immune system to prevent or treat infectious disease. The strong yet balanced, cellular and humoral immune responses that result from the immune stimulatory capacity of the nucleic acid reflect the natural defense system of the subject against invading microorganisms.

As used herein, the term "prevent", "prevented", or "preventing" and "treat", "treated" or "treating" when used with respect to the prevention or treatment of an infectious disease refers to a prophylactic treatment which increases the resistance of a subject to a microorganism or, in other words, decreases the likelihood that the subject will develop an infectious disease to the microorganism, as well as to a treatment after the subject has been infected in order to fight the infectious disease, e.g., reduce or eliminate it altogether or prevent it from becoming worse.

The CpG immunostimulatory nucleic acids are useful for treating or preventing infectious disease in a subject. A "subject" shall mean a human or vertebrate mammal including but not limited to a dog, cat, horse, cow, pig, sheep, goat, or primate, e.g., monkey. In some aspects a subject specifically excludes rodents such as mice.

In general, an antigen is administered directly to the subject by any means, such as, e.g., intravenous, intramuscular, oral, transdermal, mucosal, intranasal, intratracheal, or subcutaneous administration. The antigen can be administered systemically or locally. In some preferred embodiments, the antigen is not conjugated to the immunostimulatory nucleic acid. Administration methods are described in more detail below.

The term "substantially purified" as used herein refers to a molecular species which is substantially free of other proteins, lipids, carbohydrates or other materials with which it is naturally associated. One skilled in the art can purify polypeptides, e.g. antigens, using standard techniques for protein purification. The substantially pure polypeptide will often yield a single major band on a non-reducing polyacrylamide gel. In the case of partially glycosylated polypeptides or those that have several start codons, there may be several bands on a non-reducing polyacrylamide gel, but these will form a distinctive pattern for that polypeptide. The purity of the polypeptide can also be determined by amino-terminal amino acid sequence analysis.

The microbial antigen, if administered and if it is a polypeptide, may be in the form of a polypeptide when administered to the subject or it may be encoded by a nucleic acid vector. If the nucleic acid vector is administered to the subject the protein is expressed *in vivo.* Minor modifications of the primary amino acid sequences of polypeptide microbial antigens may also result in a polypeptide which has substantially equivalent antigenic activity, as compared to the unmodified counterpart polypeptide. Such modifications may be deliberate, as by site-directed mutagenesis, or may be spontaneous. Thus, nucleic acids having such modifications are also encompassed. When an antigen that is encoded by a nucleic acid vector is administered, the immunostimulatory nucleic acid is not the same plasmid or expression vector containing the antigen.

The nucleic acid encoding the antigen is operatively linked to a gene expression sequence which directs the expression of the protein within a eukaryotic cell. The "gene expression sequence" is any regulatory nucleotide sequence, such as a promoter sequence or promoter-enhancer combination, which facilitates the efficient transcription and translation of the protein which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, b-actin promoter and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, cytomegalovirus, the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In other aspects of the invention, the use of CpG immunostimulatory nucleic acids and oil in water emulsion allows for the administration of lower doses of antigen than could ordinarily be administered to produce an effective antigen specific immune response. Thus, the CpG immunostimulatory nucleic acids allow for the administration of lower, sub-therapeutic doses of the antigen, but with higher efficacy than would otherwise be achieved using such low doses. As one example, by administering an immunostimulatory nucleic acid with a dose of antigen that if otherwise used in combination with a conventional adjuvant such as alum would be ineffective, it is possible to achieve an effective immune response against the antigen even though one of skill in the art would not have expected that dose of antigen to provide a therapeutic benefit (i.e., a sub-therapeutic dose).

A CpG immunostimulatory nucleic acid is used in the methods of the invention. A CpG immunostimulatory nucleic acid is a nucleic acid which contains a CG dinucleotide, the C residue of which is unmethylated. CpG immimostimulatory nucleic acids are known to stimulate Th1-type immune responses. CpG sequences, while relatively rare in human DNA are commonly found in the DNA of infectious organisms such as bacteria. The human immune system has apparently evolved to recognize CpG sequences as an early warning sign of infection and to initiate an immediate and powerful immune response against invading pathogens without causing adverse reactions frequently seen with other immune stimulatory agents. Thus CpG containing nucleic acids, relying on this innate immune defense mechanism can utilize a unique and natural pathway for immune therapy. The effects of CpG nucleic acids on immune modulation have been described extensively in United States Patent No. 6,194,388, and published patent applications, such as PCT WO1996/002555, WO1998/018810, WO1998/037919, WO1998/010408, WO1998/040100, WO1999/051259, and WO1999/056755.

A CpG nucleic acid is a nucleic acid which includes at least one unmethylated CpG dinucleotide. A nucleic acid containing at least one unmethylated CpG dinucleotide is a nucleic acid molecule which contains an unmethylated cytosine in a cytosine-guanine dinucleotide sequence (i.e. "CpG DNA" or DNA containing a 5' cytosine followed by 3' guanosine and linked by a phosphate bond) and activates the immune system. The CpG nucleic acids can be double-stranded or single-stranded. Generally, double-stranded molecules are more stable *in vivo*, while single-stranded molecules have increased immune activity. Thus in some aspects of the invention it is preferred that the nucleic acid be single stranded and in other aspects it is preferred that the nucleic acid be double stranded. The terms CpG nucleic acid or CpG oligonucleotide as used herein refer to an immunostimulatory CpG nucleic acid unless otherwise indicated. The entire immunostimulatory nucleic acid can be unmethylated or portions may be unmethylated but at least the C of the 5' CG 3' must be unmethylated.

In one preferred embodiment the invention provides an immunostimulatory nucleic acid which is a CpG nucleic acid represented by at least the formula:

5'X₁X₂CGX₃X₄3'

wherein X₁, X₂, X₃, and X₄ are nucleotides. In one embodiment X₂ is adenine, guanine, cytosine, or thymine. In another embodiment X₃ is cytosine, guanine, adenine, or thymine. In other embodiments X₂ is adenine, guanine, or thymine and X₃ is cytosine, adenine, or thymine.

In another embodiment the immunostimulatory nucleic acid is an isolated CpG nucleic acid represented by at least the formula:

5'N₁X₁X₂CGX₃X₄N₂3'

wherein X₁, X₂,X₃, and X₄ are nucleotides and N is any nucleotide and N₁ and N₂ are nucleic acid sequences composed of from about 0-25 N's each. In one embodiment X₁X₂ are nucleotides selected from the group consisting of: GpT, GpG, GpA, ApA, ApT, ApG, CpT, CpA, CpG, TpA, TpT, and TpG; and X₃X₄ are nucleotides selected from the group consisting of: TpT, ApT, TpG, ApG, CpG, TpC, ApC, CpC, TpA, ApA, and CpA. Preferably X₁X₂ are GpA or GpT and X₃X₄ are TpT. In other embodiments X₁ or X₂ or both are purines and X₃ or X₄ or both are pyrimidines or X₁X₂ are GpA and X₃ or X₄ or both are pyrimidines. In another preferred embodiment X₁X₂ are nucleotides selected from the group consisting of: TpA, ApA, ApC, ApG, and GpG. In yet another embodiment X₃X₄ are nucleotides selected from the group consisting of: TpT, TpA, TpG, ApA, ApG, ApC, and CpA. X₁X₂ in another embodiment are nucleotides selected from the group consisting of: TpT, TpG, ApT, GpC, CpC, CpT, TpC, GpT and CpG.

In another preferred embodiment the immunostimulatory nucleic acid has the sequence 5'TCN₁TX₁X₂CGX₃X₄3'. The immunostimulatory nucleic acids of the invention in some embodiments include X₁X₂ selected from the group consisting of GpT, GpG, GpA and ApA and X₃X₄ is selected from the group consisting of TpT, CpT and TpC.

For facilitating uptake into cells, the immunostimulatory nucleic acids are preferably in the range of 6 to 100 bases in length. However, nucleic acids of any size greater than 6 nucleotides (even many kb long) are capable of inducing an immune response according to the invention if sufficient immunostimulatory motifs are present. Preferably the immunostimulatory nucleic acid is in the range of between 8 and 100 and in some embodiments between 8 and 50 or 8 and 30 nucleotides in size.

"Palindromic sequence" shall mean an inverted repeat (i.e., a sequence such as ABCDEE'D'C'B'A' in which A and A' are bases capable of forming the usual Watson-Crick base pairs). *In vivo,* such sequences may form double-stranded structures. In one embodiment the CpG nucleic acid contains a palindromic sequence. A palindromic sequence used in this context refers to a palindrome in which the CpG is part of the palindrome, and preferably is the center of the palindrome. In another embodiment the CpG nucleic acid is free of a palindrome. An immunostimulatory nucleic acid that is free of a palindrome is one in which the CpG dinucleotide is not part of a palindrome. Such an oligonucleotide may include a palindrome in which the CpG is not the center of the palindrome.

Exemplary immunostimulatory nucleic acid sequences include but are not limited to those immunostimulatory sequences shown in Table 1.

**Table 1**

| | |
|---|---|
| GCTAGACGTTAGCGT; | (SEQ ID NO: 1) |
| GCTAGATGTTAGCGT; | (SEQ ID NO: 2) |
| GCTAGACGTTAGCGT; | (SEQ ID NO: 3) |
| GCTAGACGTTAGCGT; | (SEQ ID NO: 4) |
| GCATGACGTTGAGCT; | (SEQ ID NO: 5) |
| ATGGAAGGTCCAGCGTTCTC; | (SEQ ID NO: 6) |
| ATCGACTCTCGAGCGTTCTC; | (SEQ ID NO: 7) |
| ATCGACTCTCGAGCGTTCTC; | (SEQ ID NO: 8) |
| ATCGACTCTCGAGCGTTCTC; | (SEQ ID NO: 9) |
| ATGGAAGGTCCAACGTTCTC; | (SEQ ID NO: 10) |
| GAGAACGCTGGACCTTCCAT; | (SEQ ID NO: 11) |
| GAGAACGCTCGACCTTCCAT; | (SEQ ID NO: 12) |
| GAGAACGCTCGACCTTCGAT; | (SEQ ID NO: 13) |
| GAGAACGCTGGACCTTCCAT; | (SEQ ID NO: 14) |
| GAGAACGATGGACCTTCCAT; | (SEQ ID NO: 15) |
| GAGAACGCTCCAGCACTGAT; | (SEQ ID NO: 16) |
| TCCATGTCGGTCCTGATGCT; | (SEQ ID NO: 17) |
| TCCATGTCGGTCCTGATGCT; | (SEQ ID NO: 18) |
| TCCATGACGTTCCTGATGCT; | (SEQ ID NO: 19) |
| TCCATGTCGGTCCTGCTGAT; | (SEQ ID NO: 20) |
| TCAACGTT; | (SEQ ID NO: 21) |
| TCAGCGCT; | (SEQ ID NO: 22) |
| TCATCGAT; | (SEQ ID NO: 23) |
| TCTTCGAA; | (SEQ ID NO: 24) |
| CAACGTT; | (SEQ ID NO: 25) |
| CCAACGTT; | (SEQ ID NO: 26) |
| AACGTTCT; | (SEQ ID NO: 27) |
| TCAACGTC; | (SEQ ID NO: 28) |
| ATGGACTCTCCAGCGTTCTC; | (SEQ ID NO: 29) |
| ATGGAAGGTCCAACGTTCTC; | (SEQ ID NO: 30) |
| ATCGACTCTCGAGCGTTCTC; | (SEQ ID NO: 31) |
| ATGGAGGCTCCATCGTTCTC; | (SEQ ID NO: 32) |
| ATCGACTCTCGAGCGTTCTC; | (SEQ ID NO: 33) |
| ATCGACTCTCGAGCGTTCTC; | (SEQ ID NO: 34) |
| TCCATGTCGGTCCTGATGCT; | (SEQ ID NO: 35) |
| TCCATGCCGGTCCTGATGCT; | (SEQ ID NO: 36) |
| TCCATGGCGGTCCTGATGCT; | (SEQ ID NO: 37) |
| TCCATGACGGTCCTGATGCT; | (SEQ ID NO: 38) |
| TCCATGTCGATCCTGATGCT; | (SEQ ID NO: 39) |
| TCCATGTCGCTCCTGATGCT; | (SEQ ID NO: 40) |
| TCCATGTCGTCCCTGATGCT; | (SEQ ID NO: 41) |
| TCCATGACGTGCCTGATGCT; | (SEQ ID NO: 42) |
| TCCATAACGTTCCTGATGCT; | (SEQ ID NO: 43) |
| TCCATGACGTCCCTGATGCT; | (SEQ ID NO: 44) |
| TCCATCACGTGCCTGATGCT; | (SEQ ED NO: 45) |
| GGGGTCAACGTTGACGGGG; | (SEQ ID NO: 46) |
| GGGGTCAGTCGTGACGGGG; | (SEQ ID NO: 47) |
| GCTAGACGTTAGTGT; | (SEQ ID NO: 48) |
| TCCATGTCGTTCCTGATGCT; | (SEQ ID NO: 49) |
| ACCATGGACGATCTGTTTCCCCTC; | (SEQ ID NO: 50) |
| TCTCCCAGCGTGCGCCAT; | (SEQ ID NO: 51) |
| ACCATGGACGAACTGTTTCCCCTC; | (SEQ ID NO: 52) |
| ACCATGGACGAGCTGTTTCCCCTC; | (SEQ ID NO: 53) |
| ACCATGGACGACCTGTTTCCCCTC; | (SEQ ID NO: 54) |
| ACCATGGACGTACTGTTTCCCCTC; | (SEQ ID NO: 55) |
| ACCATGGACGGTCTGTTTCCCCTC; | (SEQ ID NO: 56) |
| ACCATGGACGTTCTGTTTCCCCTC; | (SEQ ID NO: 57) |
| CACGTTGAGGGGCAT; | (SEQ ID NO: 58) |
| TCAGCGTGCGCC; | (SEQ ID NO: 59) |
| ATGACGTTCCTGACGTT; | (SEQ ID NO: 60) |
| TCTCCCAGCGGGCGCAT; | (SEQ ID NO: 61) |
| TCCATGTCGTTCCTGTCGTT; | (SEQ ID NO: 62) |
| TCCATAGCGTTCCTAGCGTT; | (SEQ ID NO: 63) |
| TCGTCGCTGTCTCCCCTTCTT; | (SEQ ID NO: 64) |
| TCCTGACGTTCCTGACGTT; | (SEQ ID NO: 65) |
| TCCTGTCGTTCCTGTCGTT; | (SEQ ID NO: 66) |
| TCCATGTCGTTTTTGTCGTT; | (SEQ ID NO: 67) |
| TCCTGTCGTTCCTTGTCGTT; | (SEQ ID NO: 68) |
| TCCTTGTCGTTCCTGTCGTT; | (SEQ ID NO: 69) |
| TCCTGTCGTTTTTTGTCGTT; | (SEQ ID NO: 70) |
| TCGTCGCTGTCTGCCCTTCTT; | (SEQ ID NO: 71) |
| TCGTCGCTGTTGTCGTTTCTT; | (SEQ ID NO: 72) |
| TCCATGCGTGCGTGCGTTTT; | (SEQ ID NO: 73) |
| TCCATGCGTTGCGTTGCGTT; | (SEQ ID NO: 74) |
| TCCACGACGTTTTCGACGTT; | (SEQ ID NO: 75) |
| TCGTCGTTGTCGTTGTCGTT; | (SEQ ID NO: 76) |
| TCGTCGTTTTGTCGTTTTGTCGTT; | (SEQ ID NO: 77) |
| TCGTCGTTGTCGTTTTGTCGTT; | (SEQ ID NO: 78) |
| GCGTGCGTTGTCGTTGTCGTT; | (SEQ ID NO: 79) |
| TGTCGTTTGTCGTTTGTCGTT; | (SEQ ID NO: 80) |
| TGTCGTTGTCGTTGTCGTTGTCGTT; | (SEQ ID NO: 81) |
| TGTCGTTGTCGTTGTCGTT; | (SEQ ID NO: 82) |
| TCGTCGTCGTCGTT; | (SEQ ID NO: 83) |
| TGTCGTTGTCGTT; | (SEQ ID NO: 84) |
| TCCATAGCGTTCCTAGCGTT; | (SEQ ID NO: 85) |
| TCCATGACGTTCCTGACGTT; | (SEQ ID NO: 86) |
| GTCGYT; | (SEQ ID NO: 87) |
| TGTCGYT; | (SEQ ID NO: 88) |
| AGCTATGACGTTCCAAGG; | (SEQ ID NO: 89) |
| TCCATGACGTTCCTGACGTT; | (SEQ ID NO: 90) |
| ATCGACTCTCGAACGTTCTC; | (SEQ ID NO: 91) |
| TCCATGTCGGTCCTGACGCA; | (SEQ ID NO: 92) |
| TCTTCGAT; | (SEQ ID NO: 93) |
| ATAGGAGGTCCAACGTTCTC; | (SEQ ID NO: 94) |
| GCTAGAGGGGAGGGT; | (SEQ ID NO: 95) |
| GCTAGATGTTAGGGG; | (SEQ ID NO: 96) |
| GCTAGAGGGGAGGGT; | (SEQ ID NO: 97) |
| GCTAGAGGGGAGGGT; | (SEQ ID NO: 98) |
| GCATGAGGGGGAGCT; | (SEQ ID NO: 99) |
| ATGGAAGGTCCAGGGGGCTC; | (SEQ ID NO: 100) |
| ATGGACTCTGGAGGGGGCTC; | (SEQ ID NO: 101) |
| ATGGACTCTGGAGGGGGCTC; | (SEQ ID NO: 102) |
| ATGGACTCTGGAGGGGGCTC; | (SEQ ID NO: 103) |
| ATGGAAGGTCCAAGGGGCTC; | (SEQ ID NO: 104) |
| GAGAAGGGGGGACCTTCCAT; | (SEQ ID NO: 105) |
| GAGAAGGGGGGACCTTCCAT; | (SEQ ID NO: 106) |
| GAGAAGGGGGGACCTTGGAT; | (SEQ ID NO: 107) |
| GAGAAGGGGGGACCTTCCAT; | (SEQ ID NO: 108) |
| GAGAAGGGGGGACCTTCCAT; | (SEQ ID NO: 109) |
| GAGAAGGGGCCAGCACTGAT; | (SEQ ID NO: 110) |
| TCCATGTGGGGCCTGATGCT; | (SEQ ID NO: 111) |
| TCCATGTGGGGCCTGATGCT; | (SEQ ID NO: 112) |
| TCCATGAGGGGCCTGATGCT; | (SEQ ID NO: 113) |
| TCCATGTGGGGCCTGCTGAT; | (SEQ ID NO: 114) |
| ATGGACTCTCCGGGGTTCTC; | (SEQ ID NO: 115) |
| ATGGAAGGTCCGGGGTTCTC; | (SEQ ID NO: 116) |
| ATGGACTCTGGAGGGGTCTC; | (SEQ ID NO: 117) |
| ATGGAGGCTCCATGGGGCTC; | (SEQ ID NO: 118) |
| ATGGACTCTGGGGGGTTCTC; | (SEQ ID NO: 119) |
| ATGGACTCTGGGGGGTTCTC; | (SEQ ID NO: 120) |
| TCCATGTGGGTGGGGATGCT; | (SEQ ID NO: 121) |
| TCCATGCGGGTGGGGATGCT; | (SEQ ID NO: 122) |
| TCCATGGGGGTCCTGATGCT; | (SEQ ID NO: 123) |
| TCCATGGGGGTCCTGATGCT; | (SEQ ID NO: 124) |
| TCCATGTGGGGCCTGATGCT; | (SEQ ID NO: 125) |
| TCCATGTGGGGCCTGATGCT; | (SEQ ID NO: 126) |
| TCCATGGGGTCCCTGATGCT; | (SEQ ID NO: 127) |
| TCCATGGGGTGCCTGATGCT; | (SEQ ID NO: 128) |
| TCCATGGGGTTCCTGATGCT; | (SEQ ID NO: 129) |
| TCCATGGGGTCCCTGATGCT; | (SEQ ID NO: 130) |
| TCCATCGGGGGCCTGATGCT; | (SEQ ID NO: 131) |
| GCTAGAGGGAGTGT; | (SEQ ID NO: 132) |
| GGGGGGGGGGGGGGGGGGGG; | (SEQ ID NO: 133) |
| ACTGACAGACTGACAGACTGA; | (SEQ ID NO: 134) |
| AGTGACAGACAGACACACTGA; | (SEQ ID NO: 135) |
| ACTGACAGACTGATAGACCCA; | (SEQ ID NO: 136) |
| AGTGAGAGACTGCAAGACTGA; | (SEQ ID NO: 137) |
| AATGCCAGTCCGACAGGCTGA; | (SEQ ID NO: 138) |
| CCAGAACAGAAGCAATGGATG; | (SEQ ID NO: 139) |
| CCTGAACAGAAGCCATGGATG; | (SEQ ID NO: 140) |
| GCAGAACAGAAGACATGGATG; | (SEQ ID NO: 141) |
| CCACAACACAAGCAATGGATA; | (SEQ ID NO: 142) |
| AAGCTAGCCAGCTAGCTAGCA; | (SEQ ID NO: 143) |
| CAGCTAGCCACCTAGCTAGCA; | (SEQ ID NO: 144) |
| AAGCTAGGCAGCTAACTAGCA; | (SEQ ID NO: 145) |
| GAGCTAGCAAGCTAGCTAGGA; | (SEQ ID NO: 146) |

For use in the instant invention, the CpG immunostimulatory nucleic acids may be synthesized *de novo* using any of a number of procedures well known in the art. Such compounds are referred to as "synthetic" nucleic acids. For example, the b-cyanoethyl phosphoramidite method (Beaucage, S.L., and Caruthers, M.H., Tet. Let. 22:1859, 1981); nucleoside H-phosphonate method (Garegg et al., Tet. Let. 27:4051-4054, 1986; Froehler et al., Nucl. Acid. Res. 14:5399-5407, 1986, ; Garegg et al., Tet. Let. 27:4055-4058, 1986, Gaffney et al., Tet. Let. 29:2619-2622, 1988). These chemistries can be performed by a variety of automated oligonucleotide synthesizers available in the market. These nucleic acids are referred to as synthetic nucleic acids. Alternatively, immunostimulatory nucleic acids can be produced on a large scale in plasmids, (see Sambrook, T., et al., "Molecular Cloning: A Laboratory Manual", Cold Spring Harbor laboratory Press, New York, 1989) and separated into smaller pieces or administered whole. Nucleic acids can be prepared from existing nucleic acid sequences (*e.g*., genomic or cDNA) using known techniques, such as those employing restriction enzymes, exonucleases or endonucleases. Nucleic acids prepared in this manner are referred to as isolated nucleic acids. The term "immunostimulatory nucleic acid" encompasses both synthetic and isolated CpG immunostimulatory nucleic acids.

For use *in vivo,* nucleic acids are preferably relatively resistant to degradation (e.g., are stabilized). A "stabilized nucleic acid molecule" shall mean a nucleic acid molecule that is relatively resistant to *in vivo* degradation (e.g. via an exo- or endo-nuclease). Stabilization can be a function of length or secondary structure. Immunostimulatory nucleic acids that are tens to hundreds of kbs long are relatively resistant to *in vivo* degradation. For shorter immunostimulatory nucleic acids, secondary structure can stabilize and increase their effect. For example, if the 3' end of a nucleic acid has self-complementarity to an upstream region, so that it can fold back and form a sort of stem loop structure, then the nucleic acid becomes stabilized and therefore exhibits more biological in vivo activity.

Alternatively, nucleic acid stabilization can be accomplished via backbone modifications. Preferred stabilized nucleic acids of the instant invention have a modified backbone. It has been demonstrated that modification of the nucleic acid backbone provides enhanced activity of the immunostimulatory nucleic acids when administered *in vivo.* One type of modified backbone is a phosphate backbone modification. Immunostimulatory nucleic acids, including at least two phosphorothioate linkages at the 5' end of the oligonucleotide and multiple phosphorothioate linkages at the 3' end, preferably 5, can in some circumstances provide maximal activity and protect the nucleic acid from degradation by intracellular exo- and endo-nucleases. Other phosphate modified nucleic acids include phosphodiester modified nucleic acids, combinations ofphosphodiester and phosphorothioate nucleic acids, methylphosphonate, methylphosphorothioate, phosphorodithioate, and combinations thereof. Each of these combinations in CpG nucleic acids and their particular effects on immune cells is discussed in more detail in Issued U.S. Patents 6,194,388; 6,207,646, and 6,239,116.

Although not intending to be bound by any particular theory, it is believed that these phosphate modified nucleic acids may show more stimulatory activity due to enhanced nuclease resistance, increased cellular uptake, increased protein binding, and/or altered intracellular localization.

Modified backbones such as phosphorothioates may be synthesized using automated techniques employing either phosphoramidate or H-phosphonate chemistries. Aryl-and alkyl-phosphonates can be made, e.g., as described in U.S. Patent No. 4,469,863. Alkylphosphotriesters, in which the charged oxygen moiety is alkylated as described in U.S. Patent No. 5,023,243 and European Patent No. 092,574, can be prepared by automated solid phase synthesis using commercially available reagents. Methods for making other DNA backbone modifications and substitutions have been described (Uhlmann, E. and Peyman, A., Chem. Rev. 90:544, 1990; Goodchild, J., Bioconjugate Chem. 1:165, 1990).

Both phosphorothioate and phosphodiester nucleic acids containing immunostimulatory motifs are active in immune cells. However, based on the concentration needed to induce immunostimulatory nucleic acid specific effects, the nuclease resistant phosphorothioate backbone imnunostimulatory nucleic acids are more potent than phosphodiester backbone immunostimulatory nucleic acids. For example, 2 µg/ml of the phosphorothioate has been shown to effect the same immune stimulation as a 90 µg/ml of the phosphodiester.

Another type of modified backbone, useful according to the invention, is a peptide nucleic acid. The backbone is composed of aminoethylglycine and supports bases which provide the DNA character. The backbone does not include any phosphate and thus may optionally have no net charge. The lack of charge allows for stronger DNA-DNA binding because the charge repulsion between the two strands does not exist. Additionally, because the backbone has an extra methylene group, the oligonucleotides are enzyme/protease resistant. Peptide nucleic acids can be purchased from various commercial sources, e.g., Perkin Elmer, or synthesized de novo.

Another class of backbone modifications include 2'-O-methylribonucleosides (2'-Ome). These types of substitutions are described extensively in the prior art and in particular with respect to their immunostimulating properties in Zhao et aL, Bioorganic and Medicinal Chemistry Letters, 1999, 9:24:3453. Zhao et al. describes methods of preparing 2'-Ome modifications to nucleic acids.

The nucleic acid molecules of the invention may include naturally-occurring or synthetic purine or pyrimidine heterocyclic bases as well as modified backbones. Purine or pyrimidine heterocyclic bases include, but are not limited to, adenine, guanine, cytosine, thymidine, uracil, and inosine. Other representative heterocyclic bases are disclosed in US Patent No. 3,687,808, issued to Merigan, et al. The terms "purines" or "pyrimidines" or "bases" are used herein to refer to both naturally-occurring or synthetic purines, pyrimidines or bases.

Other stabilized nucleic acids include non-ionic DNA analogs, such as alkyl- and aryl-phosphates (in which the charged phosphonate oxygen is replaced by an alkyl or aryl group), phosphodiester and alkylphosphotriesters, in which the charged oxygen moiety is alkylated. Nucleic acids which contain diol, such as tetraethyleneglycol or hexaethyleneglycol, at either or both termini have also been shown to be substantially resistant to nuclease degradation.

The immunostimulatory nucleic acids having backbone modifications useful according to the invention in some embodiments are S- or R-chiral immunostimulatory nucleic acids. An "S chiral immunostimulatory nucleic acid" as used herein is an immunostimulatory nucleic acid wherein at least two nucleotides have a backbone modification forming a chiral center and wherein at least 75% of the chiral centers have S chirality. An "R chiral immunostimulatory nucleic acid" as used herein is an immunostimulatory nucleic acid wherein at least two nucleotides have a backbone modification forming a chiral center and wherein at least 75% of the chiral centers have R chirality. The backbone modification may be any type of modification that forms a chiral center. The modifications include but are not limited to phosphorothioate, methylphosphonate, methylphosphorothioate, phosphorodithioate, 2'-Ome and combinations thereof.

The chiral immunostimulatory nucleic acids must have at least two nucleotides within the nucleic acid that have a backbone modification. All or less than all of the nucleotides in the nucleic acid, however, may have a modified backbone. Of the nucleotides having a modified backbone (referred to as chiral centers), at least 75% of the have a single chirality, S or R. Thus, less than all of the chiral centers may have S or R chirality as long as at least 75% of the chiral centers have S or R chirality. In some embodiments at least 80,%, 85%, 90%, 95%, or 100% of the chiral centers have S or R chirality. In other embodiments at least 80%, 85%, 90%, 95%, or 100% of the nucleotides have backbone modifications.

The S- and R- chiral immunostimulatory nucleic acids may be prepared by any method known in the art for producing chirally pure oligonucleotides. Stec et al teach methods for producing stereopure phosphorothioate oligodeoxynucleotides using an oxathiaphospholane. (Stec, W.J., et al., 1995, J. Am. Chem. Soc., 117:12019). Other methods for making chirally pure oligonucleotides have been described by companies such as ISIS Pharmaceuticals. US Patents which disclose methods for generating stereopure oligonucleotides include 5883237, 5837856, 5599797, 5512668, 5856465, 5359052, 5506212, 5521302 and 5212295.

As used herein, administration of an immunostimulatory nucleic acid is intended to embrace the administration of one or more imnunostimulatory nucleic acids which may or may not differ in terms of their profile, sequence, backbone modifications and biological effect. As an example, CpG nucleic acids and poly-G nucleic acids may be administered to a single subject. In another example, a plurality of CpG nucleic acids which differ in nucleotide sequence may also be administered to a subject.

The therapeutic formulations of the invention are oil-in-water emulsions. As used herein the term oil-in-water emulsion refers to a fluid composed of a heterogeneous mixture of minute drops of oil suspended in water. Oil-in-water emulsions are well known in the art. One preferred oil-in-water emulsion is sold under the trademark name EMULSIGEN^{™} (sold by MPV Laboratories, Nebraska, U.S.A).

The term "effective amount" of an immunostimulatory nucleic acid refers to the amount necessary or sufficient to realize a desired biologic effect. For example, an effective amount of an immunostimulatory nucleic acid could be that amount necessary to cause activation of the immune system, resulting potentially in the development of an antigen specific immune response. According to some aspects of the invention, an effective amount is that amount of an immunostimulatory nucleic acid and that amount of a therapeutic formulation, which when combined or co-administered, results in a synergistic response to the cancer or infectious agent, either in the prevention or the treatment of the cancer or infectious disease. A synergistic amount is that amount which produces a response that is greater than the sum of the individual effects of either the immunostimulatory nucleic acid and the therapeutic formulation alone. For example, a synergistic combination of a CpG immunostimulatory nucleic acid and a therapeutic formulation provides a biological effect which is greater than the combined biological effect which could have been achieved using each of the components (i.e., the nucleic acid and the medicament) separately.

One of ordinary skill in the art can empirically determine the effective amount of a particular immunostimulatory nucleic acid and therapeutic formulation combination without necessitating undue experimentation. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is entirely effective to treat the particular subject.

In some embodiments, the CpG immunostimulatory nucleic acids are administered in an effective amount to stimulate or induce a Th1 immune response, or a Th2 immune response, or a general immune response. An effective amount to stimulate a Th1 immune response may be defined as that amount which stimulates the production of one or more Th1-type cytokines such as interleukin 2 (IL-2), IL-12, tumor necrosis factor (TNFα) and interferon gamma (IFN-y), and/or production of one or more Thl-type antibodies. An effective amount to stimulate a Th2 immune response, on the other hand, may be defined as that amount which stimulates the production of one or more Th2-type cytokines such as IL-4, IL-5, IL-6, IL-9, IL-10 and IL-13, and/or the production of one or more Th2-type antibodies.

Immunostimulatory nucleic acids are known to be useful for preventing bacterial and viral infections.

In some instances, a sub-therapeutic dosage of the antigen is used in the treatment of a subject having, or at risk of developing, cancer or infectious disease. As an example, it has been discovered according to the invention, that when the antigen is used together with the immunostimulatory nucleic acid, the antigen can be administered in a sub-therapeutic dose and still produce a desirable therapeutic result. A "sub-therapeutic dose" as used herein refers to a dosage which is less than that dosage which would produce a therapeutic result in the subject if administered in the absence of the other agent. Thus, the sub-therapeutic dose of an antigen is one which, alone or in combination with a conventional adjuvant such as alum, would not produce the desired therapeutic result in the subject in the absence of the administration of the immunostimulatory nucleic acid. Therapeutic doses of antigens are well known in the field of vaccination. These dosages have been extensively described in references relied upon by the medical profession as guidance for vaccination. Therapeutic dosages of immunostimulatory nucleic acids have also been described in the art and methods for identifying therapeutic dosages in subjects are described in more detail herein.

For any compound described herein a therapeutically effective amount can be initially determined from cell culture assays. In particular, the effective amount of immunostimulatory nucleic acid can be determined using in vitro stimulation assays. The stimulation index of the immunostimulatory nucleic acid can be compared to that of previously tested immunostimulatory acids. The stimulation index can be used to determine an effective amount of the particular oligonucleotide for the particular subject, and the dosage can be adjusted upwards or downwards to achieve the desired levels in the subject.

Therapeutically effective amounts can also be determined in animal studies. For instance, the effective amount of immunostimulatory nucleic acid and therapeutic formulation to induce a synergistic response can be assessed using in vivo assays of tumor regression and/or prevention of tumor formation. Relevant animal models include assays in which malignant cells are injected into the animal subjects, usually in a defined site. Generally, a range of immunostimulatory nucleic acid doses are administered into the animal along with a range of therapeutic formulation doses. Inhibition of the growth of a tumor following the injection of the malignant cells is indicative of the ability to reduce the risk of developing a cancer. Inhibition of further growth (or reduction in size) of a pre-existing tumor is indicative of the ability to treat the cancer. Mice which have been modified to have human immune system elements can be used as recipients of human cancer cell lines to determine the effective amount of the synergistic combination.

A therapeutically effective dose can also be determined from human data for immunostimulatory nucleic acids which have been tested in humans (human clinical trials have been initiated) and for compounds which are known to exhibit similar pharmacological activities, such as other adjuvants, e.g., LT and other antigens for vaccination purposes.

The applied dose of both the immunostimulatory nucleic acid and the therapeutic formulation can be adjusted based on the relative bioavailability and potency of the administered compounds, including the adjuvants used. Adjusting the dose to achieve maximal efficacy based on the methods described above and other methods are well within the capabilities of the ordinarily skilled artisan.

Subject doses of the compounds described herein typically range from about 0.1 µg to 10,000 mg, more typically from about 1 µg/day to 8000 mg, and most typically from about 10 µg to 100 µg. Stated in terms of subject body weight, typical dosages range from about 0.1 µg to 20 mg/kg/day, more typically from about 1 to 10 mg/kg/day, and most typically from about 1 to 5 mg/kg/day.

In other embodiments of the invention, the CpG immunostimulatory nucleic acid is administered on a routine schedule. A "routine schedule" as used herein, refers to a predetermined designated period of time. The routine schedule may encompass periods of time which are identical or which differ in length, as long as the schedule is predetermined. For instance, the routine schedule may involve administration of the immunostimulatory nucleic acid on a daily basis, every two days, every three days, every four days, every five days, every six days, a weekly basis, a monthly basis or any set number of days or weeks there-between, every two months, three months, four months, five months, six months, seven months, eight months, nine months, ten months, eleven months, twelve months, etc. Alternatively, the predetermined routine schedule may involve administration of the immunostimulatory nucleic acid on a daily basis for the first week, followed by a monthly basis for several months, and then every three months after that. Any particular combination would be covered by the routine schedule as long as it is determined ahead of time that the appropriate schedule involves administration on a certain day.

The CpG immunostimulatory nucleic acids may be delivered to the subject in the form of a plasmid vector. In some embodiments, one plasmid vector could include both the immunostimulatory nucleic acid and a nucleic acid encoding an antigen. In other embodiments, separate plasmids could be used. In yet other embodiments, no plasmids could be used.

The CpG immunostimulatory nucleic acid and the therapeutic formulation may be administered alone (e.g. in saline or buffer) or using any delivery vectors known in the art. For instance the following delivery vehicles have been described: cochleates (Gould-Fogerite et al., 1994, 1996); Emulsomes (Vancott et al., 1998, Lowell et al., 1997); ISCOMs (Mowat et al., 1993, Carlsson et al., 1991, Hu et., 1998, Morein et al., 1999); liposomes (Childers et al., 1999, Michalek et al., 1989, 1992, de Haan 1995a, 1995b); live bacterial vectors (e.g., *Salmonella, Escherichia coli, Bacillus calmatte guerin, Shigella, Lactobacillus*) (Hone et al.., 1996, Pouwels et al., 1998, Chatfield et al., 1993, Stover et al., 1991, Nugent et al., 1998); live viral vectors (e.g., Vaccinia, adenovirus, Herpes Simplex) (Gallichan et al., 1993, 1995, Moss et al., 1996, Nugent et al., 1998, Flexner et al., 1988, Morrow et al., 1999); microspheres (Gupta et al., 1998, Jones et al., 1996, Maloy et al., 1994, Moore et al., 1995, O'Hagan et al., 1994, Eldridge et al., 1989); nucleic acid vaccines (Fynan et al., 1993, Kuklin et al., 1997, Sasaki et al., 1998, Okada et al., 1997, Ishii et al., 1997); polymers (e.g. carboxymethylcellulose, chitosan) (Hamajima et al., 1998, Jabbal-Gill et al., 1998); polymer rings (Wyatt et al., 1998); proteosomes (Vancott et al., 1998, Lowell et al., 1988, 1996, 1997); sodium fluoride (Hashi et al., 1998); transgenic plants (Tacket et al., 1998, Mason et al., 1998, Haq et al., 1995); virosomes (Gluck et al., 1992, Mengiardi et al., 1995, Cryz et al., 1998); and, virus-like particles (Jiang et al., 1999, Leibl et al., 1998).

The CpG immunostimulatory nucleic acid may be combined with additional therapeutic agents such as cytokines to enhance immune responses even further. The immunostimulatory nucleic acid and other therapeutic agent may be administered simultaneously or sequentially. When the other therapeutic agents are administered simultaneously they can be administered in the same or separate formulations, but are administered at the same time. The administration of the other therapeutic agents and the immunostimulatory nucleic acid may also be temporally separated, meaning that the therapeutic agents are administered at a different time, either before or after, the administration of the immunostimulatory nucleic acid. The separation in time between the administration of these compounds may be a matter of minutes or it may be longer. Other therapeutic agents include but are not limited to cytokines, immunotherapeutic antibodies, antigens, etc.

Immune responses can also be induced or augmented by the co-administration or colinear expression of cytokines or co-stimulatory molecules with the immunostimulatory nucleic acids. The cytokines may be administered directly with immunostimulatory nucleic acids or may be administered in the form of a nucleic acid vector that encodes the cytokine, such that the cytokine can be expressed *in vivo.* In one embodiment, the cytokine is administered in the form of a plasmid expression vector. The term "cytokine" is used as a generic name for a diverse group of soluble proteins and peptides which act as humoral regulators at nano- to picomolar concentrations and which, either under normal or pathological conditions, modulate the functional activities of individual cells and tissues. These proteins also mediate interactions between cells directly and regulate processes taking place in the extracellular environment. Cytokines also are central in directing the T cell response. Examples of cytokines include, but are not limited to IL-1, IL-2, IL-4, IL-5, IL-6, IL-7, IL-10, IL-12, IL-15, IL-18, granulocyte-macrophage colony stimulating factor (GM-CSF), granulocyte colony stimulating factor (G-CSF), interferon-γ (IFN-γ), IFN-α, tumor necrosis factor (TNF), TGF-β, FLT-3 ligand, and CD40 ligand. In some embodiments, the cytokine is a Th1 cytokine. In still other embodiments, the cytokine is a Th2 cytokine. In other embodiments a cytokine is not administered in combination with the immunostimulatory nucleic acid.

In other aspects, the disclosure relates to kits. One kit of the invention includes a container housing an immunostimulatory nucleic acid and a container housing an oil-in-water emulsion and instructions for timing of administration of the immunostimulatory nucleic acid and the oil-in-water emulsion. Another kit of the invention includes a container housing an immunostimulatory nucleic acid and instructions for timing of administration of the immunostimulatory nucleic acid. Optionally the kit may also include an antigen, housed in a separate container or formulated with the immunostimulatory nucleic acid or therapeutic formulation. Optionally the antigen may be in a sustained release device. A sustained release vehicle is used herein in accordance with its prior art meaning of any device which slowly releases the antigen.

Such systems can avoid repeated administrations of the compounds, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono-di- and triglycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

The oil-in-water-emulsion is housed in at least one container. The container may be a single container housing all of the formulation together or it may be multiple containers or chambers housing individual dosages, such as a blister pack. The kit also has instructions for timing of administration of the therapeutic formulation. The instructions would direct the subject having cancer or at risk of cancer to take the therapeutic formulation at the appropriate time. For instance, the appropriate time for delivery of the medicament may be as the symptoms occur. Alternatively, the appropriate time for administration of the medicament may be on a routine schedule such as monthly or yearly.

The pharmaceutical compositions of the disclosure contain an effective amount of an immunostimulatory nucleic acid and therapeutic formulation optionally included in a pharmaceutically-acceptable carrier. The term "phamiaceutically-acceptable carrier" means one or more compatible solid or liquid filler, dilutants or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The immunostimulatory nucleic acid and therapeutic formulation may be administered per se (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions. Another suitable compound for sustained release delivery is GELFOAM, a commercially available product consisting of modified collagen fibers.

Alternatively, the active compounds may be in powder form for constitution with a suitable vehicle, e.g., sterile pyrogen-free water, before use.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

The immunostimulatory nucleic acid and therapeutic formulations can be administered on fixed schedules or in different temporal relationships to one another. The various combinations have many advantages over the prior art methods.

Immunostimulatory nucleic acid and therapeutic formulation may be administered by any ordinary route for administering medications. Depending upon the type of disorder to be treated, immunostimulatory nucleic acids and therapeutic formulations may be inhaled, ingested or administered by systemic routes. Systemic routes include oral and parenteral. Inhaled medications are preferred in some embodiments because of the direct delivery to the lung, particularly in the treatment of respiratory disease or lung cancer. Several types of metered dose inhalers are regularly used for administration by inhalation. These types of devices include metered dose inhalers (MDI), breath-actuated MDI, dry powder inhaler (DPI), spacer/holding chambers in combination with MDI, and nebulizers. Preferred routes of administration include but are not limited to oral, parenteral, intramuscular, intranasal, intratracheal, intrathecal, intravenous, inhalation, ocular, vaginal, and rectal.

For use in therapy, an effective amount of the immunostimulatory nucleic acid and therapeutic formulation can be administered to a subject by any mode that delivers the nucleic acid to the affected organ or tissue, or alternatively to the immune system. "Administering" the pharmaceutical composition of the present invention may be accomplished by any means known to the skilled artisan. Preferred routes of administration include but are not limited to oral, parenteral, intramuscular, subcutaneous, intranasal, intratracheal, inhalation, ocular, vaginal, and rectal.

For oral administration, the compounds (i.e., immunostimulatory nucleic acids, therapeutic formulations, and the other therapeutic agents) may be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers for neutralizing internal acid conditions or may be administered without any carriers.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For administration by inhalation, the compounds for use according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit maybe determined by providing a valve to deliver a metered amount Capsules and cartridges of e.g. gelatin for use in an inhaler or insufflator may be formulated containing a powder mix of the compound and a suitable powder base such as lactose or starch. Techniques for preparing aerosol delivery systems are well known to those of skill in the art. Generally, such systems should utilize components which will not significantly impair the biological properties of the therapeutic, such as the immunostimulatory capacity of the nucleic acids (see, for example, Sciarra and Cutie, "Aerosols," in Remington's Pharmaceutical Sciences, 18th edition, 1990, pp 1694-1712). Those of skill in the art can readily determine the various parameters and conditions for producing aerosols without resort to undue experimentation.

The compounds, when it is desirable to deliver them systemically, may be formulated for parenteral administration by injection, *e.g*., by bolus injection or continuous infusion. Formulations for injection may be presented in unit dosage form, *e.g.*, in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

In still other embodiments of the invention, the immunostimulatory nucleic acids are provided in the intravenous solutions, bags and/or tubing used to deliver transfusions into cancer patients. The immunostimulatory nucleic acids may be introduced into an intravenous solution which is administered to the subject prior to receiving the transfusion, or it may be introduced into the blood transfusion itself (i.e., the suspension of red blood cells or platelets). Alternatively, the intravenous bags and tubing maybe themselves be coated on their internal surfaces with immunostimulatory nucleic acids, or they may be impregnated with immunostimulatory nucleic acids during manufacture. Methods for manufacture of intravenous systems for the delivery of biologically active materials are known in the art. Examples include those described in U.S.P. Nos.: 4,973,307, and 5,250,028, issued to Alza, Corp.

The compounds may also be formulated in rectal or vaginal compositions such as suppositories or retention enemas, *e.g.*, containing conventional suppository bases such as cocoa butter or other glycerides.

In addition to the formulations described previously, the compounds may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer, Science 249: 1527-1533, 1990.

The present invention is further illustrated by the following Examples, which in no way should be construed as further limiting.

### Example 1: CpG in combination with EMULSIGEN^{™}:

The experiments were performed to test the immunogenicity and protective efficacy of a bovine herpesvirus-1 (BHV-1) subunit vaccine co-adjuvanted with EMULSIGEN^{™} (Em) and a CpG ODN in cattle. A truncated version of BHV-1 glycoprotein D (tgD) co-adjuvanted with Em and CpG ODN at concentrations of 25, 2.5 or 0.25 mg/dose produced a stronger and more balanced Th1/Th2 immune response, higher serum neutralization antibodies and greater protection following BHV-1 challenge, compared to tgD adjuvanted with VSA3, Em, or CpG ODN alone. Furthermore, tgD co-adjuvanted with Em and 25 mg of a non-CpG ODN/dose produced comparable levels of immunity to Em alone and lower than the CpG ODN/Em combinations.

### Materials and Methods

Cells and Virus: Strains P8-2 and 108 of BHV-1 were propagated in Madin Darby bovine kidney (MDBK) cells as described previously ***(***van Drunen Littel-van den Hurk, S., J. et al 1994. A subunit gIV vaccine, produced by transfected mammalian cells in culture, induces mucosal immunity against bovine herpesvirus-1 in cattle. Vaccine 12:1295-1302*.).* Strain 108 was used for challenging animals, whereas for stimulation of in vitro proliferation of PBMC, strain P8-2 was used.

Production, processing and purification of BHV-1 tgD: A truncated version of BHV-1 gD (tgD) was constructed by terminating the protein at amino acid 355, immediately upstream of the transmembrane anchor. It was expressed in MDBK cells under regulation of the bovine heat shock 70A (hsp70) gene promoter *(*Kowalski, J et al 1993. Heat-shock promoter-driven synthesis of secreted bovine herpesvirus glycoproteins in transfected cells. Vaccine 11:1100-1107*).* Truncated gD was produced, processed and purified as described elsewhere ***(***van Drunen Littel-van den Hurk, S., J. et al 1994. A subunit gIV vaccine, produced by transected mammalian cells in culture, induces mucosal immunity against bovine herpesvirus-1 in cattle. Vaccine 12:1295-1302*.).*

CpG and non-CpG ODN: Unmethylated CpG dinucleotides in a synthetic oligodeoxynucleotide (ODN) preparation (Qiagen, Hilden, Germany) were used either as adjuvant or as co-adjuvant in this study. The CpG ODN used was ODN 2007 (TCGTCGTTGTCGTTTTGTCGTT; CpG motifs are underlined). To determine whether immune responses were induced by the CpG dinucleotides, we also used a non-CpG ODN; 2041 (CTGGTCTTTCTGGTTTTTTTCTGG) (Qiagen). The CpG and non-CpG ODN were phosphorothioate modified to increase resistance to nuclease degradation ***(***Kuhnle, G., A. et al. 1998. The class II membrane glycoprotein G of bovine respiratory syncytial virus, expressed from a synthetic open reading frame, is incorporated into virions of recombinant bovine herpesvirus 1. J.Virol. 72:3804-3811*).*

Immunization: Eight groups of seven, nine month-old, BHV-1-seronegative Angus and Hereford cross calves were immunized subcutaneously with 50 µg BHV-1 tgD adjuvanted with either 30% vol/vol EMULSIGEN^{™} (Em) (MVP Laboratories, Nebraska, U.S.A), 30% vol/vol VSA3 (Em containing 24 mM dimethyldioctadecylammonium bromide [DDA]), 25 mg of CpG ODN (CpG), a combination of 30% Em and 25 (high), 2.5 (medium) or 0.25 (low) mg CpG ODN (H CpG/Em, M CpG/Em, L CpG/Em respectively), or with a combination of Em and 25 mg non-CpG ODN (non-CpG/Em). The vaccines were administered subcutaneously in a 2 ml volume. A placebo group of calves was immunized with 2 ml PBS only. Thirty-nine days later, the animals were re-immunized and then challenged 2 weeks after the secondary immunization (Day 53 of vaccination).

Experimental challenge and clinical evaluation: Five weeks after secondary immunization, animals were transported into an isolation pen weighed and examined clinically. The calves were then individually exposed for 4 min to an aerosol of 10⁷ PFU of BHV-1 as previously described *(*Loelir, B.L, et al. 2000. Gene gun-mediated DNA immunization primes development of mucosal immunity against bovine herpesvirus 1 in cattle. J. Virol. 74:6077-6086*;* van Drunen Littel-van den Hurk, S., et al. 1990. Epitope specificity of the protective immune response induced by individual bovine herpesvirus-1 glycoproteins. Vaccine 8:358-368*).* Following challenge, the calves were weighed daily. Furthermore, they were clinically evaluated for 11 consecutive days. Clinical evaluation was performed at the same time each day by a veterinarian who was blind to the vaccine status of the animals. The clinical signs evaluated included fever (rectal temperatures >40°C), depression, rhinitis, and conjuctivitis.

Sampling and virus isolation: Animals were bled for assessment of antibody responses on days 0, 14, 39, 47, 53, 57, 61, 64 and 67 after vaccination. Blood with anticoagulant (ethylenediamine tetraacetic acid [EDTA] to a final concentration of 0.2%) was collected on days 50 and 61 for assessment of *in vitro* proliferation and IFN-γ production by ELISPOT and ELISA assays. Nasal tampons containing up to 5 ml of nasal fluid were collected every second day post challenge and processed the same day to measure virus shedding. Virus recovered from nasal tampons was quantified by plaque titration in microtitre plates with an antibody overlay as previously described **(**Rouse, B.T. and L.A. Babiuk. 1974. Host responses to infectious bovine rhinotracheitis virus. III. Isolation and immunologic activities of bovine T lymphocytes. J.Immunol. 113:1391-1398*).*

Enzyme-linked immunosorbent assay (ELISA): In order to determine specific antibody responses before and after challenge, 96 well polystyrene microtiter plates (Immulon 2, Dynatech, Gaithersburg, Md) were coated overnight with 0.05 µg per well of either purified tgD or purified tgB per well (Li, Y., et al. 1996. Production and characterization of bovine herpesvirus 1 glycoprotein B ectodomain derivatives in an hsp70A gene promoter-based expression system. Arch Virol. 141:2019-2029). Serially diluted bovine sera, starting at 1:10 in threefold dilutions, were incubated for 2 hours at room temperature. Alkaline phosphatase (AP)-conjugated goat anti-bovine IgG (Kirkegaard & Perry Laboratories, Gaithersburg, Md) at a dilution of 1:5,000 was used to detect bound IgG. The reaction was visualized with *p*-nitrophenyl phosphate (Sigma Chemical Co., Oakville, Ontario, Canada).

Immunoglobulin isotypes using enzyme-linked immunosorbent assay: In order to determine the specific IgG1 and IgG2 antibody responses of cattle immunized with tgD, polystyrene microtiter plates were coated overnight with 0.05 µg of purified tgD per well and blocked for 30 min at 37°C with 1% heat inactivated horse serum. Serially diluted bovine sera, starting at 1:10 in threefold dilutions, were incubated overnight at 4°C. Bound antibodies were detected with monoclonal antibodies against bovine IgG1 (M-23) or IgG2 (M-37) at dilutions of 1:40,000 and 1:8000 respectively, which in turn were detected with AP-conjugated goat anti-mouse IgG (Kirkegaard & Perry Laboratories, Gaithersburg, Md) at a dilution of 1:10,000. The reaction was visualized as for ELISA assays. Results were expressed as ratios of IgG1 to IgG2.

Virus neutralization assays: The neutralization titres of the bovine sera were determined as described previously (Babiuk, L.A., et al. 1975. Defence mechanisms against bovine herpesvirus: relationship of virus-host cell events to susceptibility to antibody-compliment cell lysis. Infect.Immun. 12:958-963*).* The titers were expressed as the reciprocal of the highest dilution of antibody that caused a 50% reduction of plaques relative to virus control.

*In Vitro* Proliferation of PBMC: Peripheral blood mononuclear cells (PBMC) were isolated on Ficoll-Plaque PLUS (Pharmacia, Mississauga, Ontario, Canada) and cultured in triplicate in a 96 well tissue culture plate at 3.5 x 10⁵ cells/well in minimum essential medium (Gibco BRL, Grand Island N.Y, U.S.A) supplemented with 10% (vol/vol) fetal bovine serum (Sigma Chemical Co), 2mM L-glutamine (Gibco-BRL), 500 mg/ml gentamicin, 5 x 10⁻⁵ M 2-mercaptoethanol and 1 mg/ml dexamethasone. Cells were stimulated with gD at a final concentration of 1 µg/ml. Control cells were unstimulated. After 72 hours in culture, the cells were pulsed with [*methyl*-³H] thymidine (Amersham, Oakville, Ontario, Canada) at a concentration of 0.4 µCu/well. The cells were harvested 18 h later using a semiautomatic cell harvester (Skatron, Starling VA, U.S.A) and radioactivity was determined by scintilation counting. Proliferative responses were calculated as the means of triplicate wells and expressed as a stimulated index (SI) where SI represents counts per min in the presence of antigen divided by counts per min in the absence of antigen.

ELISPOT assays: Nitrocellulose plates (Whatman, New Jersey, U.S.A) were coated overnight at 4°C with a bovine interferon-gamma (IFN-γ)-specific monoclonal antibody at a dilution of 1:400. Unbound antibody was washed off with 0.05% vol/vol PBS-Tween-20 (PBS-T) with a final wash in PBS. PBMC were isolated as for proliferation assays and cultured at 10⁶ cells/well in the presence of gD at a final concentration of 0.4 µg/ml. Control cells were cultured with media only. After 24 h, the cells were washed, resuspended in culture medium, transferred to nitrocellulose plates and incubated for a further 24 h at 37°C, after which cells were washed off with 0.05% vol/vol PBS-T. Subsequently, the plates were incubated for 2 h at RT with rabbit polyclonal antibodies against bovine IFN-γ at a dilution of 1:100 and then for 2 h at RT with biotinylated rat anti-rabbit IgG (Zymed, San Francisco, CA, U.S.A), followed by streptavidin-AP (GIBCO-BRL, Ontario, Canada), each at 1:1000 dilution. Bound IFN-γ was visualized using bromochloroindolyl phosphate/nitro-blue tetrazolium (BCIP/NBT) substrate tablets (Sigma Chemical Co). The plates were washed in distilled water and air dried, after which stained spots were counted under 400 x magnification. The number of IFN-γ-secreting cells was expressed as the difference between the number of spots per 10⁶ cells in gD-stimulated wells and the number of spots per 10⁶ cells in control wells.

IFN-γ ELISA: Bovine PBMC were cultured as for ELISPOT assays. After 24 h, the supernatants were harvested and serially diluted in 96 well plates coated with monoclonal antibodies against bovine IFN-γ. Purified bovine IFN-γ of known concentration was used as a standard. The standard curve ranged from 2000 to 7.8 pg/ml (r > 0.98). Samples and standards were assayed at eight 2-fold dilutions in PBS-T at 100 µl/well. Bound IFN-γ was detected using rabbit anti-IFN-γ IgG, which was in turn detected using AP-conjugated goat anti-rabbit IgG. The reaction was visualized as described for tgD-specific antibody ELISAs. The absorbance of the substrate was measured at 405 and 490 nm. An ELISA reader program (Microplate manager 5, BIO RAD Laboratories, Ontario, Canada) was used to construct a standard curve and to compute the concentration of IFN-γ in the samples.

Statistical analysis: To allow for unequal distribution, all data were transformed by log transformation prior to performance of statistical analysis. Differences in serum neutralization titers, isotype ratios, *in vitro* proliferative responses, ELISPOT and IFN-γ ELISA data were investigated using one-way analysis of variance and Tukey's multiple comparison test. Differences in the number of animals with signs of disease among vaccine groups (temperature increase, weight loss and virus shedding) and between tgD and tgB-specific antibodies in bovine serum before and after challenge, were determined by the two-way analysis of variance and the Tukey honestly significantly different (HSD) multiple comparison test.

### Results

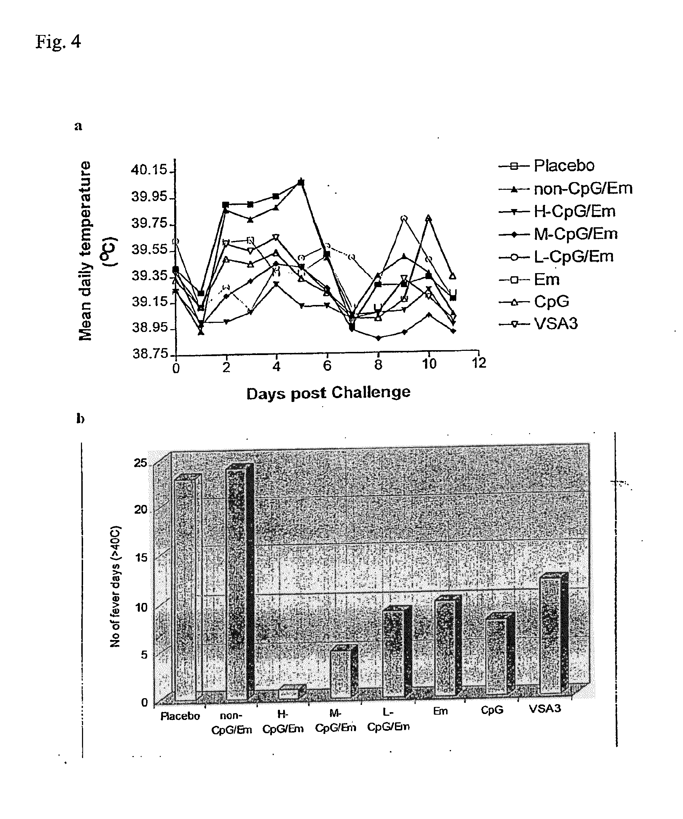

Humoral immune responses to tgD: In order to assess the adjuvant capabilities of CpG ODN, BHV-1 tgD was adjuvanted with 25 mg/dose CpG, Em or VSA3, or co-adjuvanted with Em and CpG at concentrations of 25, 2.5 or 0.25 mg/dose (H-, M- or L-CpG/Em), or with Em and 25 mg/dose of a non-CpG ODN (non-CpG/Em). With the exception of the VSA3 group, all vaccinated groups had significantly higher levels of neutralizing antibodies than the placebo group fourteen days following the primary immunization (p<0.001) (Figure 4). Antibody levels in the H-CpG/Em group were significantly (p<0.001) higher than those of the non-CpG/Em, Em, CpG or VSA3 groups. The antibody levels increased dramatically after secondary immunization such that on day 47 all three CpG/Em groups had significantly (p<0.001) higher titers than all other vaccine groups. This data provides evidence that the concentration of CpG ODN in the vaccines had no significant effect on the secondary immune response. Importantly, antibody titers of animals immunized with tgD co-adjuvanted with non-CpG/Em, were not significantly different from the titers of the Em group. In addition, the titers in the non-CpG /Em group were not significantly different from those of the CpG and VSA3 groups.

To determine the type of immune response generated, tgD-specific IgG1 and IgG2 antibodies in bovine serum were determined and IgG1:IgG2 ratios were measured 8 days after secondary immunization. The ratios were similar both after primary immunization and after challenge. A balanced immune response (~1:1 ratio) was measured in the three CpG /Em groups and the CpG group, there was no statistical difference between these groups. In contrast, the Em, VSA3 and non-CpG/Em formulated vaccines produced an IgG1-biased immune response (>1600:1). The non-CpG/Em group produced a higher IgG1:IgG2 ratio than did the L-CpG/Em group. However, the non-CpG/Em group was significantly (p<0.05) different from both the M-CpG/Em and H-CpG/Em groups. There were no significant differences between the three CpG/Em groups nor between the non-CpG/Em and Em groups. In addition, all three CpG/Em groups were significantly different from both the Em (p<0.001) and VSA3 (p<0.01) groups.

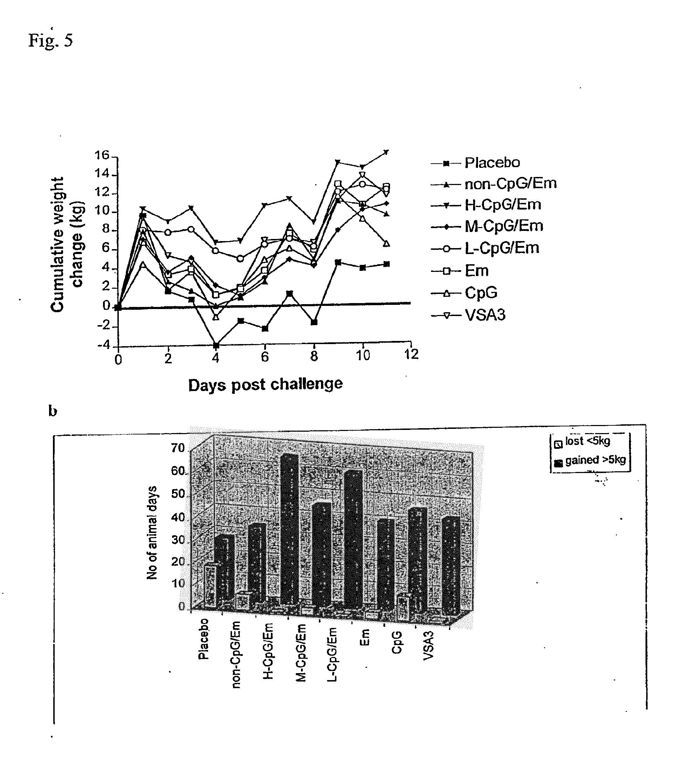

Cell-mediated immune responses to tgD: To examine cell-mediated immunity induced by the vaccinations, *in vitro* proliferative responses of bovine lymphocytes to BHV-1 gD were measured. Although the proliferative responses before challenge tended to be stronger in the CpG/Em vaccinated animals than in animals vaccinated with non-CpG/Em, CpG, or Em, the difference was not statistically significant (Figure 5a). However, the proliferative responses in the H-CpG/Em and L-CpG/Em groups were significantly (p<0.05) higher than those in the VSA3 and placebo groups. To further confirm T-cell activation, production of IFN-γ was assessed. Although the numbers of IFN-γ secreting cells in the CpG/Em groups were not dependent on the concentration of CpG ODN used, they were significantly higher (p<0.001) than the number of IFN-γ secreting cells in the non-CpG/Em, Em, VSA3 and placebo groups (Figure 5b). Following BHV-1 challenge, proliferative responses of PBMC groups were ~2-fold stronger in the CpG/Em than in other vaccinated groups (Figure 5b). In contrast there was no difference between the CpG/Em groups and the CpG group. The amount of IFN-γ measured before challenge in the supernatant of cultured PBMC of vaccinated animals followed a similar pattern of response to that of the ELISPOT (Figure 5c). The CpG/Em groups were not significantly different from each other nor from the CpG or Em groups. However, the amount of IFN-γ measured in these groups was significantly higher than that measured in the placebo (p<0.01), non-CpG/Em (p<0.05) and VSA3 (p<0.01) groups. These data confirm the ability of CpG ODN even when combined with EMULSIGEN^{™} to induce Thl-type immune responses.

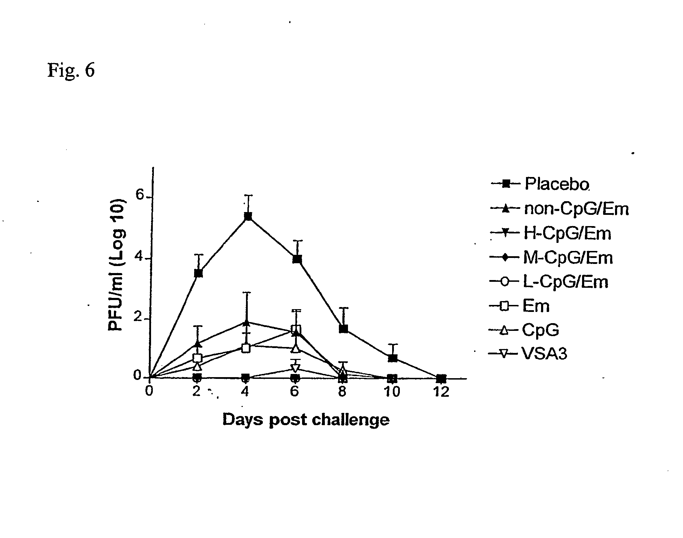

Immune responses after BHV-1 infection: An increase in the level of either serum neutralizing antibodies or antibodies specific for viral proteins after challenge is another indication of infection. Although all groups were seronegative to BHV-1 glycoprotein B (tgB) prior to challenge, antibodies against tgB in the placebo, CpG, Em, VSA3 and non-CpG/Em groups, but not in the CpG/Em groups, increased significantly (p<0.01) after challenge (Figure 6b). Serum neutralizing titers (Figure 4) and antibodies against tgD (Figure 6a) in the placebo, CpG, Em, VSA3 and non-CpG/Em groups also increased significantly (p<0.005) after challenge suggesting that these groups were not entirely protected from BHV-1 infection. Although the M-CpG/Em group also exhibited some increase in both the level of serum neutralizing antibodies and antibodies against tgD after challenge, these increases were not significant. Serum neutralizing titers and antibodies against tgD in the H-CpG/Em actually decreased after challenge, while those in the M-CpG/Em group remained stable. These results suggest the induction of sterile immunity in the CpG- EMULSIGEN^{™} formulations.

Protection from challenge with BHV-1: All animals were healthy prior to challenge. After challenge, the mean rectal temperature increased from day 2 to day 6 in all but the M-and H-CpG/Em groups, where the temperature remained ≤ 39.5°C over the entire follow up period (Figure 7). The placebo and non-CpG/Em groups, exhibited the greatest increase in temperature (to ~40.1°C by day 6) and were significantly different from the M- and-H-CpG/Em groups (p<0.002). Although temperatures of calves in the L-CpG/Em group increased steadily to 39.5°C by day 6 and the temperatures were significantly different (p=0.006) from those of the H-CpG/Em group, they were also different from the placebo group (p<0.001), but not from the M-CpG/Em group. The mean rectal temperature in the Em, CpG and VSA3 groups also increased to >39.4°C on days 2 to day 4 after which it fell to <39°C.

Another assessment of morbidity is the extent of weight loss following BHV-1 infection. Whereas animals in the H- and L-CpG/Em groups experienced minimal or no loss in weight over the course of the trial, those in other groups experienced weight loss of up to 8 kg 4 days after challenge (Figure 8).

To further determine the level of protection from BHV-1 infection, the extent of shedding from the nasal passages was assessed. Whereas animals in the CpG, Em, and non-CpG/Em groups began shedding virus on day 2 after challenge and continued to do so at least until day 8, no virus was recovered from the nasal tampons of animals in either of the CpG/Em groups (Figure 9). Although the vaccinations had a significant (p<0.001) effect on virus shedding, the three CpG/Em groups were statistically different from only the non-CpG/Em (p=0.003) and placebo (p<0.001) groups.

## Claims

1. A composition, for administration to cattle, comprising an immunostimulatory nucleic acid, an oil-in-water emulsion, and a bovine herpesvirus-1 (BHV-1) antigen; wherein the immunostimulatory nucleic acid is a CpG oligonucleotide; and wherein the BHV-1 antigen is truncated BHV-1 glycoprotein D.

2. The composition according to claim 1 wherein said CpG oligonucleotide is selected from the group consisting of:
SEQ ID Nos 1 to 94 and 138,
2007 (TCGTCGTTGTCGTTTTGTCGTT);
2142 (TCGCGTGCGTTTTGTCGTTTTGACGTT);
2135 (TCGTCGTTTGTCGTTTTGTCGTT); and
2216 (ggGGGACGATCGTCgggggG).

3. The composition according to claim 2 wherein said CpG oligonucleotide is
2007 (TCGTCGTTGTCGTTTTGTCGTT);
2142 (TCGCGTGCGTTTTGTCGTTTTGACGTT);
2135 (TCGTCGTTTGTCGTTTTGTCGTT); or
2216 (ggGGGACGATCGTCgggggG).

4. The composition of any one of claims 1 to 3 wherein the immunostimulatory nucleic acid has a modified backbone.

5. The composition of claim 4 wherein the modified backbone is a phosphate modified backbone.

6. The composition of claim 5, wherein the phosphate modified backbone is a phosphorothioate modified backbone.

7. The composition of claim 4, wherein the modified backbone is a peptide modified oligonucleotide backbone.

8. A composition according to any one of claims 1 to 7 for use as a medicament.

9. Use of a composition according to any one of claims 1 to 7 for the manufacture of a medicament for reducing viral shedding in a cow.

10. Use of a composition according to any one of claims 1 to 7 for the manufacture of a medicament for the prevention and/or treatment of a BHV-1 infection in a cow.

11. Use according to claim 10 wherein said antigen of said composition is in a sub-therapeutic dosage.

12. Use according to claim 11 wherein the sub-therapeutic dose of the antigen is a dose which is at least 50% less than a minimal effective dose of antigen for producing an antigen specific immune response when the antigen is formulated with alum.

13. Use according to claim 11, wherein the sub-therapeutic dose of the antigen is a dose which is at least 90% less than a minimal effective dose of antigen for producing an antigen specific immune response when the antigen is formulated with alum.

14. Use according to any one of claims 8 to 13 wherein said composition is administered on a routine schedule.

15. Use according to claim 14, wherein the composition is administered on a weekly basis.

16. Use according to claim 15 wherein the composition is administered on a daily basis.

17. Use according to claim 16, wherein the composition is administered on a monthly basis.

18. Use according to any one of claims 8 to 17, wherein the composition is administered orally.

19. Use according to any one of claims 8 to 17, wherein the composition is administered by injection.

20. Use according to claim 19 wherein the composition is injected sub-cutaneously.

21. Use according to any one of claims 8 to 17, wherein the composition is administered through a sustained release device.

22. Use according to any one of claims 8 to 17 wherein the cow is an immunocompromised subject.

## Patentansprüche

1. Zusammensetzung zur Verabreichung an Rinder, die eine immunstimulierende Nucleinsäure, eine Öl-in-Wasser-Emulsion und ein Bovines Herpesvirus-1 (BHV-1) Antigen umfasst, wobei die immunstimulierende Nucleinsäure ein CpG-Oligonucleotid ist, und wobei das BHV-1-Antigen ein verkürztes BHV-1-Glykoprotein-D ist.

2. Zusammensetzung nach Anspruch 1, wobei das CpG-Oligonucleotid ausgewählt ist aus der Gruppe bestehend aus:
SEQ ID NOs:1 bis 94 und 138,
2007 (TCGTCGTTGTCGTTTTGTCGTT);
2142 (TCGCGTGCGTTTTGTCGTTTTGACGTT);
2135 (TCGTCGTTTGTCGTTTTGTCGTT); und
2216 ggGACGATCGTCgggggG).

3. Zusammensetzung nach Anspruch 2, wobei das CpG-Oligonucleotid
2007 (TCGTCGTTGTCGTTTTGTCGTT);
2142 (TCGCGTGCGTTTTGTCGTTTTGACGTT);
2135 (TCGTCGTTTGTCGTTTTGTCGTT); oder
2216 ggGACGATCGTCgggggG) ist.

4. Zusammensetzung nach einem der Ansprüche 1 bis 3, wobei die immunstimulierende Nucleinsäure ein modifiziertes Rückgrat hat.

5. Zusammensetzung nach Anspruch 4, wobei das modifizierte Rückgrat ein Phosphat-modifiziertes Rückgrat ist.

6. Zusammensetzung nach Anspruch 5, wobei das Phosphat-modifizierte Rückgrat ein Phosphorothioat-modifiziertes Rückgrat ist.

7. Zusammensetzung nach Anspruch 4, wobei das modifizierte Rückgrat ein Peptid-modifiziertes Oligonucleotid-Rückgrat ist.

8. Zusammensetzung nach einem der Ansprüche 1 bis 7 zur Verwendung als Medikament.

9. Verwendung einer Zusammensetzung nach einem der Ansprüche 1 bis 7 zur Herstellung eines Medikaments zur Reduzierung viraler Freisetzung in einem Rind.

10. Verwendung einer Zusammensetzung nach einem der Ansprüche 1 bis 7 zur Herstellung eines Medikaments zur Vorbeugung und/oder Behandlung einer BHV-1-Infektion in einem Rind.

11. Verwendung nach Anspruch 10, wobei das Antigen der Zusammensetzung in einer subtherapeutischen Dosierung ist.

12. Verwendung nach Anspruch 11, wobei die subtherapeutische Dosis des Antigens eine Dosis ist, die mindestens 50% geringer ist als eine minimale wirksame Dosis des Antigens zum Auslösen einer Antigen-spezifischen Immunantwort, wenn das Antigen mit Alaun formuliert ist.

13. Verwendung nach Anspruch 11, wobei die subtherapeutische Dosis des Antigens eine Dosis ist, die mindestens 90% geringer ist als eine minimale wirksame Dosis des Antigens zum Auslösen einer Antigen-spezifischen Immunantwort, wenn das Antigen mit Alaun formuliert ist.

14. Verwendung nach einem der Ansprüche 8 bis 13, wobei die Zusammensetzung nach einem routinemäßigen Verabreichungsplan verabreicht wird.

15. Verwendung nach Anspruch 14, wobei die Zusammensetzung wöchentlich verabreicht wird.

16. Verwendung nach Anspruch 15, wobei die Zusammensetzung täglich verabreicht wird.

17. Verwendung nach Anspruch 16, wobei die Zusammensetzung monatlich verabreicht wird.

18. Verwendung nach einem der Ansprüche 8 bis 17, wobei die Zusammensetzung oral verabreicht wird.

19. Verwendung nach einem der Ansprüche 8 bis 17, wobei die Zusammensetzung durch Injektion verabreicht wird.

20. Verwendung nach Anspruch 19, wobei die Zusammensetzung subkutan injiziert wird.

21. Verwendung nach einem der Ansprüche 8 bis 17, wobei die Zusammensetzung durch eine Retard-Vorrichtung verabreicht wird.

22. Verwendung nach einem der Ansprüche 8 bis 17, wobei das Rind ein immunsupprimiertes Individuum ist.

## Revendications

1. Composition, destinée à être administrée à du bétail, comprenant un acide nucléique immunostimulateur, une émulsion huile-dans-eau, et un antigène de l'herpèsvirus bovin de type 1 (BHV-1) ; dans laquelle l'acide nucléique immunostimulateur est un oligonucléotide CpG ; et dans laquelle l'antigène du BHV-1 est une glycoprotéine D du BHV-1 tronquée.

2. Composition selon la revendication 1, dans laquelle ledit oligonucléotide CpG est sélectionné dans le groupe constitué de :
SEQ ID NO: 1 à 94 et 138,
2007 (TCGTCGTTGTCGTTTTGTCGTT) ;
2142 (TCGCGTGCGTTTTGTCGTTTTGACGTT) ;
2135 (TCGTCGTTTGTCGTTTTGTCGTT) ; et
2216 (ggGGGACGATCGTCgggggG).

3. Composition selon la revendication 2, dans laquelle ledit oligonucléotide CpG est
2007 (TCGTCGTTGTCGTTTTGTCGTT) ;
2142 (TCGCGTGCGTTTTGTCGTTTTGACGTT) ;
2135 (TCGTCGTTTGTCGTTTTGTCGTT) ; ou
2216 (ggGGGACGATCGTCgggggG).

4. Composition selon l'une quelconque des revendications 1 à 3, dans laquelle l'acide nucléique immunostimulateur possède un squelette modifié.

5. Composition selon la revendication 4, où le squelette modifié est un squelette modifié par un phosphate.

6. Composition selon la revendication 5, où le squelette modifié par un phosphate est un squelette modifié par un phosphorothioate.

7. Composition selon la revendication 4, où le squelette modifié est un squelette oligonucléotidique modifié par un peptide.

8. Composition selon l'une quelconque des revendications 1 à 7 destinée à être utilisée en tant que médicament.

9. Utilisation d'une composition selon l'une quelconque des revendications 1 à 7 pour la fabrication d'un médicament destiné à réduire l'excrétion virale chez une vache.

10. Utilisation d'une composition selon l'une quelconque des revendications 1 à 7 pour la fabrication d'un médicament destiné à la prévention et/ou au traitement d'une infection par le BHV-1 chez une vache.

11. Utilisation selon la revendication 10, dans laquelle ledit antigène de ladite composition est en une dose sous-thérapeutique.

12. Utilisation selon la revendication 11, dans laquelle la dose sous-thérapeutique de l'antigène est une dose qui est au moins 50 % inférieure à une dose minimale efficace d'antigène pour produire une réponse immunitaire spécifique à l'antigène lorsque l'antigène est formulé avec de l'alun.

13. Utilisation selon la revendication 11, dans laquelle la dose sous-thérapeutique de l'antigène est une dose qui est au moins 90 % inférieure à une dose minimale efficace d'antigène pour produire une réponse immunitaire spécifique à l'antigène lorsque l'antigène est formulé avec de l'alun.

14. Utilisation selon l'une quelconque des revendications 8 à 13, dans laquelle ladite composition est administrée selon un calendrier systématique.

15. Utilisation selon la revendication 14, dans laquelle la composition est administrée chaque semaine.

16. Utilisation selon la revendication 15, dans laquelle la composition est administrée chaque jour.

17. Utilisation selon la revendication 16, dans laquelle la composition est administrée chaque mois.

18. Utilisation selon l'une quelconque des revendications 8 à 17, dans laquelle la composition est administrée par voie orale.

19. Utilisation selon l'une quelconque des revendications 8 à 17, dans laquelle la composition est administrée par injection.

20. Utilisation selon la revendication 19, dans laquelle la composition est injectée par voie sous-cutanée.

21. Utilisation selon l'une quelconque des revendications 8 à 17, dans laquelle la composition est administrée par l'intermédiaire d'un dispositif à libération prolongée.

22. Utilisation selon l'une quelconque des revendications 8 à 17, dans laquelle la vache est un sujet immunodéprimé.
